# EUROPEAN PATENT APPLICATION

(11) **EP 4 523 701 A2**
(43) Date of publication of application: **19.03.2025**
(21) Application number: 23802958.1
(22) Date of filing: 10.05.2023
(51) Int. Cl.: A61K 39/155, A61P 31/14, C12N 7/01, C07K 14/135, A61K 39/235, C12N 15/861

(54) **ADENOVIRAL VECTOR VACCINE AGAINST RESPIRATORY SYNCYTIAL VIRUS, AND PREPARATION METHOD THEREFOR AND USE THEREOF**

(30) Priority: 10.05.2022 CN 202210504179
(71) Applicant: Cansino Biologics Inc., Tianjin 300457 (CN)
(72) Inventor: YANG, Zening, Tianjin 300457 (CN); SHAO, Juan, Tianjin 300457 (CN); SUI, Xiuwen, Tianjin 300457 (CN); TANG, Xue, Tianjin 300457 (CN); WANG, Haomeng, Tianjin 300457 (CN); ZHAO, Jiayu, Tianjin 300457 (CN); CAO, Longlong, Tianjin 300457 (CN); XI, Zhiai, Tianjin 300457 (CN); WU, Dan, Tianjin 300457 (CN); ZHU, Tao, Tianjin 300457 (CN)
(74) Representative: Finnegan Europe LLP
(86) International application number: PCT/CN2023/093287
(87) International publication number: WO 2023/217189

(57) **Abstract**

The present disclosure provides a respiratory syncytial virus-based adenovirus vector vaccine, a method for preparing same, and use thereof. Specifically, an adenovirus vector suitable for a transmucosal formulation against RSV is selected from a variety of adenovirus vectors. The vaccine is administered by inhalation, which can simulate the virus infection process, stimulate the immune response, and avoid local adverse effects such as injection site pain and the like due to intramuscular injection. By means of inhalation, the atomized vaccine can finally reach the lung through the respiratory tract, thus inducing the respiratory tract mucosal immunity while stimulating the humoral immunity and cellular immunity. The stimulation of the pulmonary mucosal immunity is the most effective method for preventing RSV infection and spread. The vaccine stimulates the lung mucosal immunity and exhibits a well induced humoral immunity level. The present disclosure screens the dose for inhalation and the selected dose is lower compared with the that of the intramuscular injection, thus possessing better safety.

## Description

### TECHNICAL FIELD

The present disclosure relates to a transmucosal formulation, a method for preparing same, and use thereof, and specifically, to a transmucosal formulation for preventing and/or treating RSV infection.

### BACKGROUND

Respiratory syncytial virus (RSV) is a highly contagious virus that is prevalent throughout the world for a long period of time. It causes pulmonary and respiratory infections in all age groups, particularly infants, the elderly, and the immunosuppressed population.

After respiratory syncytial virus (RSV) was discovered in 1950, it soon became a well known pathogen associated with lower and upper respiratory tract infections in humans with two major subtypes A and B. Worldwide, about 34 million children aged 5 or younger suffer from RSV infection annually, leading to about 3.4 million hospitalizations and 160 to 200 thousand deaths. The risk of infection is higher in the elderly, with approximately 3%-7% of the elderly aged over 60 years being infected with RSV each year. The elderly are at higher risk of developing severe disease due to decreased immunity and underlying diseases. RSV causes enormous economic losses to the world each year and is a serious threat to human health. Over 3 million people are hospitalized for RSV infection globally each year, resulting in nearly 60 thousand deaths.

The development of RSV vaccines started since the first discovery of RSV in humans in 1957. The first product entering the clinical stage in the world is the formalin-inactivated RSV vaccine of Pfizer. In a number of clinical studies conducted, the RSV vaccine failed to protect the vaccinee, but on the contrary, led to an enhanced respiratory disease (ERD, that is, the vaccine does not prevent the vaccinee from RSV infection, but may worsen a potential RSV infection in the vaccinee). In one study, 16 out of 20 infants (80%) in the test group were hospitalized with severe conditions, and two infants died. Thus, the US FDA ceased the clinical studies of all RSV vaccines, and the RSV vaccine development experienced a long silence period. In the subsequent 60 years, the scientific community has not stopped the research on RSV vaccines and has found that the ERD effect of the formalin-inactivated RSV vaccine is probably due to the activation of Th2-CD4+ T cells by the vaccine and the occurrence of pneumonia mediated by related cytokines. The reveal of the mechanism of ERD and the progress of vaccine preparation techniques renewed the research and development of RSV vaccines, which, however, is still challenging.

For example, the RSV F protein recombinant nanoparticle vaccine (ResVax) developed by Novavax failed to reach the primary and secondary endpoints in its phase III clinical trial. Another RSV vaccine candidate GSK3003891A from GSK has completed the phase I clinical study and delivered preliminary results, but the phase II study was discontinued in 2017 soon after the start.

Compared with the convention intramuscular injections, the use of transmucosal immune formulations for vaccines was rarely reported, and particularly, no research data on transmucosal formulations for RSV are present. Although transmucosal immune formulations may have many advantages as described above, this delivery mode may reduce the residence time of the antigen in the mucosa and limit the delivery efficiency of the antigen and thus the application of transmucosal immune formulations. In addition, different immunogens have significant specificity, different carriers/delivery modes may lead to significant differences in the deposition of the drug in the lung, and the drugs deposited at different sites may result in different absorption rates, thereby directly impacting the vaccine efficacy.

In order to fill the technical gaps, overcome various problems in the prior art, and further improve the immune efficacy of pneumonia vaccines, the inventors developed a transmucosal formulation against RSV.

### SUMMARY

The present disclosure provides a composition for resisting respiratory syncytial virus (RSV) infection.

Unless otherwise defined, all scientific and technical terms used in the present disclosure have the same meaning as commonly understood by those skilled in the art to which the present disclosure relates.

The term "recombinant" as used herein implies that the virus has been artificially modified. For example, the actively cloned ends have been altered and/or the virus comprises a heterologous gene, that is, the virus is not a native wild-type adenovirus.

The term "recombinant adenovirus" as used herein is based on the adenovirus as used herein and is, at least in sequence, derived from the wild type. Such a recombinant adenovirus can be achieved by molecular cloning using the wild-type genome or parts thereof as the starting material. Known sequences of the wild-type adenovirus genome may also be used to regenerate (parts of) the genome by DNA synthesis, which can be performed by commercial service companies in the field of DNA synthesis and/or molecular cloning (e.g., GeneArt, Invitrogen, GenScripts, and Eurofins) using conventional procedures.

Those of ordinary skill should appreciate that many different polynucleotides and nucleic acids may encode the same polypeptide due to the degeneracy of the genetic codes. It should also be understood that those of ordinary skill can, using conventional techniques, make nucleotide substitutions that do not affect the polypeptide sequence encoded by the polynucleotides described herein, thereby reflecting the codon usage of any particular host organism in which the polypeptide is to be expressed. Thus, unless otherwise specified, the "nucleotide sequence encoding an amino acid sequence" includes all nucleotide sequences that are degenerate versions of each other and that encode the same amino acid sequence. Nucleotide sequences encoding proteins and RNAs may comprise introns.

As used herein, the term "fragment" refers to a sequence having an amino- and/or carboxylterminal and/or an internal deletion. It should be appreciated that for the purpose of inducing an immune response and generally for the purpose of vaccination, the protein does not need to be full-length or have all the functions of a wild type, and a fragment of the protein may be equally applicable.

As used herein, the term "composition" refers to a vaccine or other composition prepared from a recombinant adenovirus that can express the target gene by using an adenovirus as a vector and recombining the target gene into the adenovirus genome; or it refers to a pharmaceutical composition, and therefore generally comprises a pharmaceutically acceptable diluent, carrier, or excipient. It may or may not comprise other active ingredients. In some certain examples, it may be a combined vaccine that further comprises additional components that induce an immune response (e.g., other proteins against RSV and/or against other infectious agents).

As used herein, the term "simian adenovirus" refers to an adenovirus of a non-human simian species, such as rhesus macaque, cynomolgus macaque, gorilla, vervet monkey, baboon, bonnbo, chimpanzee, etc.

In the present disclosure, the composition comprises an effective amount of a fragment encoding an RSV antigen. Also provided is a method for preventing a severe lower respiratory tract disease caused by RSV infection and replication and leading to hospitalization and reducing the frequency of complications (such as pneumonia and bronchiolitis) caused by RSV infection and replication in a subject. Therefore, the present disclosure also provides a method for preventing or reducing a severe lower respiratory tract disease caused by RSV, preventing or reducing (e.g., shortening) the hospitalization caused by RSV, and/or reducing the frequency and/or severity of pneumonia or bronchiolitis caused by RSV in a subject.

The present disclosure screens various adenovirus vectors for respiratory syncytial virus, and provides a composition for resisting respiratory syncytial virus (RSV) infection, comprising a recombinant adenovirus vector, wherein a gene encoding an RSV antigen is inserted into the recombinant adenovirus vector, and the composition is a transmucosal formulation; preferably, the composition is a vaccine.

Specifically, the adenovirus vector comprises a human adenovirus and/or a simian adenovirus vector; preferably, the human adenovirus is Ad5, Ad26, Ad35, or Ad48, and the simian adenovirus vector is sAd36 or sAd37; more preferably, the recombinant adenovirus is preferably Ad5, Ad26, or sAd36.

The fusion protein F and attachment protein G are the keys for the virus to invade human body, and both can be ideal targets for virus development. The F protein has two conformations: an active conformation (pre-F form before fusion with the host cell) and an inactive conformation (post-F after fusion with the host cell). The pre-F form has six major antigenic sites (Ø, I, II, III, IV, and V), while the post-F from has four sites (I, II, III, and IV). The pre-F conformation possesses higher antigenicity and is selected in the present disclosure.

Specifically, the present disclosure encodes an RSV F protein that is stabilized in the prefusion conformation (prefusion RSV F protein).

Specifically, the gene encoding the RSV antigen is based on one or more of the RSV F protein of the prefusion conformation (pre-F), the attachment protein G, and/or the small hydrophobic (SH) protein.

The specific nucleic acid of the RSV fusion proteins F, the attachment protein G, or the small hydrophobic (SH) proteins can be retrieved by techniques well known to those skilled in the art. As is conventional in the art, the sequences are provided herein in the 5' to 3' direction.

Specifically, in some certain examples, the nucleic acid encoding the RSV F (pre-F) protein comprises the amino acid sequence set forth in SEQ ID NO: 1.

In some certain examples, the nucleic acid encoding the RSV F (pre-F) protein comprises the amino acid sequence set forth in SEQ ID NO: 2.

In some certain examples, the nucleic acid encoding the RSV F (pre-F) protein comprises the amino acid sequence set forth in SEQ ID NO: 3.

In some certain examples, the nucleic acid encoding the RSV F/G proteins comprises the amino acid sequence set forth in SEQ ID NO: 4.

In some certain examples, the nucleic acid encoding the RSV F (pre-F) protein comprises the amino acid sequence set forth in SEQ ID NO: 5.

In some certain examples, the nucleic acid encoding the RSV F (pre-F) protein comprises the amino acid sequence set forth in SEQ ID NO: 6.

In some certain examples, the nucleic acid encoding the RSV F (pre-F) and G proteins is codonoptimized for expression in a human cell.

Specifically, the transmucosal formulation is an aerosol inhalation formulation, a nasal drop or spray formulation, or a dry powder inhalation formulation; preferably, the aerosol inhalation formulation is an oral aerosol inhalation formulation or a nasal aerosol inhalation formulation. Preferably, the dosage form is an aerosol inhalant, and the vaccine is atomized by the aerosol inhalation device to form particles with a size of 10 µm or less, preferably 0.5 to 10 µm, and more preferably 5 to 10 µm.

Specifically, the gene encoding the RSV antigen may be derived from a native RSV strain or any recombinant RSV strain, preferably from a human RSV strain, such as the A2, Long, or B strain. Specifically, the composition comprises a pharmaceutically acceptable excipient, and the excipient includes but is not limited to one or more of the following: a buffer, a protectant, a stabilizer, a surfactant, an osmotic pressure regulator, a preservative, an inactivator, and/or a human albumin.

Specifically, the dose of the recombinant adenovirus in the transmucosal formulation is 1 × 10⁷ to × 10¹⁰ IFU/dose, preferably 1 × 10⁷ to 6 × 10⁹ IFU/dose, and more preferably 1 × 10⁷ to 1.3 × 10⁹ IFU/dose.

Specifically, the unit dose of the formulation is 0.05-5 mL, preferably 0.1-1 mL.

The present disclosure provides a method for preparing a composition, comprising: propagating the recombinant adenovirus in a host cell culture, isolating and purifying the recombinant adenovirus, and formulating the recombinant adenovirus into a pharmaceutically acceptable transmucosal formulation.

Specifically, the recombinant adenovirus lacks at least a portion of the E1 region and/or the E3 region of the adenovirus genome.

Specifically, the recombinant adenovirus lacks at least a portion of the E4 region and/or the E2 region of the adenovirus genome.

The present disclosure provides a kit for preventing and/or treating respiratory syncytial virus infection, comprising the transmucosal formulation and a delivery device.

Specifically, the delivery device is an aerosol inhalation device, preferably an aerosol generator. Specifically, the vaccine is atomized by the aerosol inhalation device to form particles with a size of 10 µm or less, preferably 0.5 to 10 µm, and more preferably 5 to 10 µm.

The present disclosure provides use of a composition in preparing a vaccine for inducing an immune response in a mammal, wherein the immunization is a prime immunization or a boost immunization.

Specifically, a sufficient amount of the composition is administered to the subject, and the mammal has generated a primary immune response against the same immunogen before the use of the composition.

Specifically, the composition is administered in one dose, two doses, or three doses. Specifically, the composition may be administered in combination with an immunological composition of a recombinant adenovirus of an identical or different serotype, and/or, in combination with a composition of a protein expressed by an RSV antigenic nucleic acid, and/or,
in combination with an adjuvant, and/or,
in combination with an additional therapeutic agent;

Preferably, the additional therapeutic agent may be selected from, but is not limited to, a therapeutic agent against RSV infection, such as a standard therapeutic agent against lower respiratory tract RSV infection during hospitalization, a bronchodilator, and an antibiotic, including (but not limited to) epinephrine, an anticholinergic, an antipyretic, and/or a nonsteroid anti-inflammatory drug.

The present disclosure has the following beneficial effects:
1. In one aspect of the present disclosure, an adenovirus vector suitable for a transmucosal formulation against RSV was selected from a variety of adenovirus vectors and administered by inhalation, which can simulate the virus infection process and stimulate the immune response. The transmucosal formulation of the present disclosure has good compliance, and can avoid local adverse effects such as injection site pain and the like due to intramuscular injection.
2. By means of inhalation, the atomized vaccine of the present disclosure can finally reach the lung through the respiratory tract, thus inducing the respiratory tract mucosal immunity while stimulating the humoral immunity and cellular immunity. The stimulation of the pulmonary mucosal immunity may be the most effective method for preventing the infection and spread. The vaccine stimulates the lung mucosal immunity and exhibits a well induced humoral immunity level. After the body is immunized, neutralizing antibodies and specific IgGs against the original strain may be produced. The antibodies can be favorably transmitted from a mother to the fetus via the placenta through the placenta blood barrier, thus possessing great effects on preventing the infection in babies in a short term after birth.
3. The present disclosure screened doses for inhalation and achieved selected dose that is lower compared with that of the intramuscular injection with improved safety. The invention described herein can produce triple effects of humoral immunity, cellular immunity, and mucosal immunity.
4. The transmucosal formulation described herein can be used for the prime immunization or the boost immunization.

### BRIEF DESCRIPTION OF THE DRAWINGS

FIG. 1 illustrates cells infected by recombinant viruses sAd36-rsv-F, Ad26-rsv-F, Ad5-rsv-F, sAd37-rsv-F, Ad35-rsv-F, and Ad48-rsv-F;
FIG. 2 illustrates cells infected by recombinant viruses sAd36-rsv-F/G, Ad26-rsv-F/G, and Ad5-rsv-F/G;

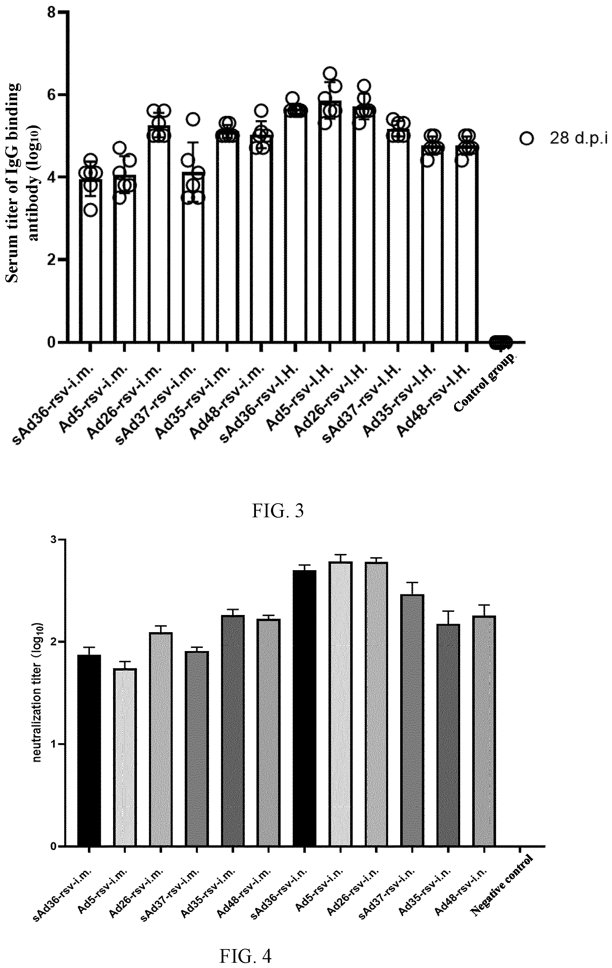

FIG. 3 illustrates the serum titer of IgG binding antibody against pre-F in mice 28d after the immunization with transmucosal formulations or injections of sAd36-rsv-F/Ad26-rsv-F/Ad5-rsv-F/sAd37-rsv-F/Ad35-rsv-F/Ad48-rsv-F;
FIG. 4 illustrates the serum titer of neutralizing antibody against RSV A2 virus in mice 28d after the immunization with transmucosal formulations or injections prepared from sAd36-rsv-F/Ad26-rsv-F/Ad5-rsv-F/sAd37-rsv-F/Ad35-rsv-F/Ad48-rsv-F;

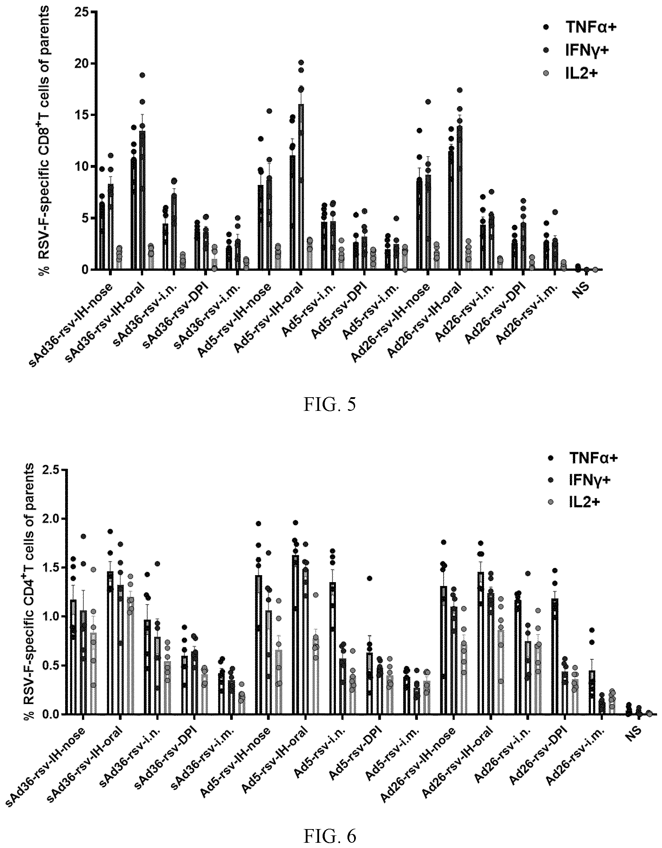

FIG. 5 illustrates the TNF-α, IFN-γ, and IL-2 response levels of SPL CD8+ T cell 28d after mice were immunized with a transmucosal formulation or injection prepared from sAd36-rsv-F/Ad26-rsv-F/Ad5-rsv-F;
FIG. 6 illustrates the TNF-α, IFN-γ, and IL-2 response levels of SPL CD4+ T cell 28d after mice were immunized with a transmucosal formulation or injection prepared from sAd36-rsv-F/Ad26-rsv-F/Ad5-rsv-F;

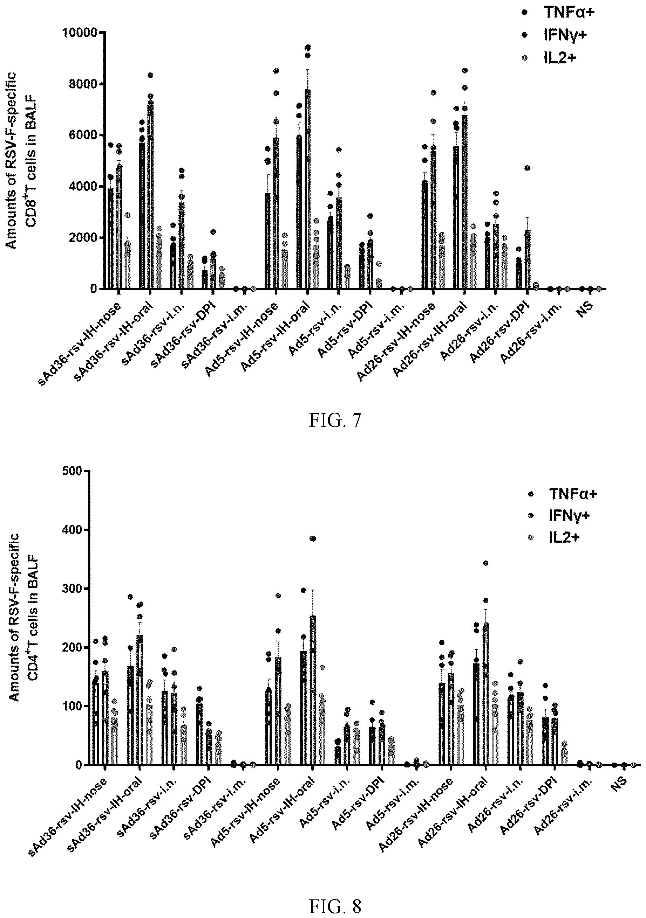

FIG. 7 illustrates the TNF-α, IFN-y, and IL-2 response levels of BALF CD8+ T cell 28d after mice were immunized with a transmucosal formulation or injection prepared from sAd36-rsv-F/Ad26-rsv-F/Ad5-rsv-F;
FIG. 8 illustrates the TNF-α, IFN-y, and IL-2 response levels of BALF CD4+ T cell 28d after mice were immunized with a transmucosal formulation or injection prepared from sAd36-rsv-F/Ad26-rsv-F/Ad5-rsv-F;

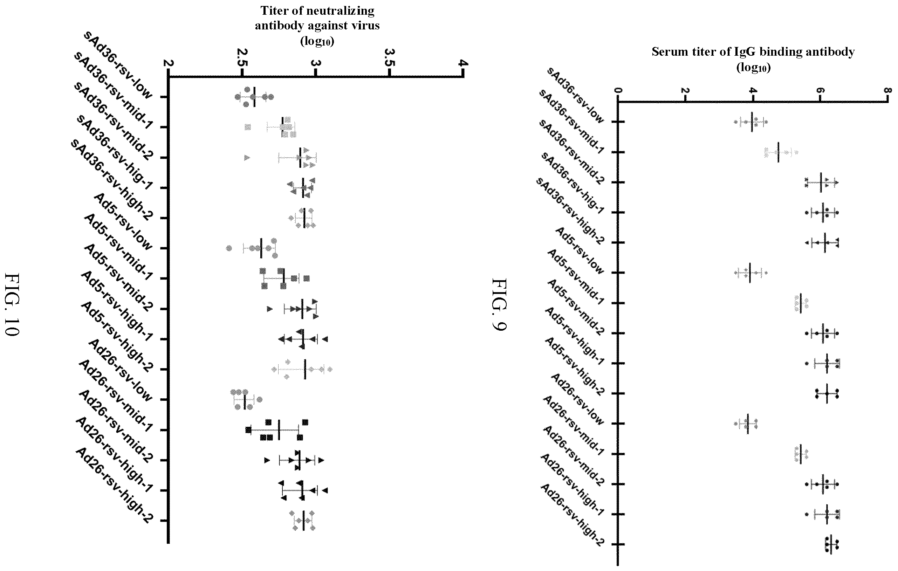

FIG. 9 illustrates the serum titer of IgG binding antibody 28 days after mice were immunized with different doses of sAd36-rsv-F/Ad26-rsv-F/Ad5-rsv-F transmucosal formulation;
FIG. 10 illustrates the serum titer of neutralizing antibody against RSV A2 virus 28 days after mice were immunized with different doses of sAd36-rsv-F/Ad26-rsv-F/Ad5-rsv-F transmucosal formulation;

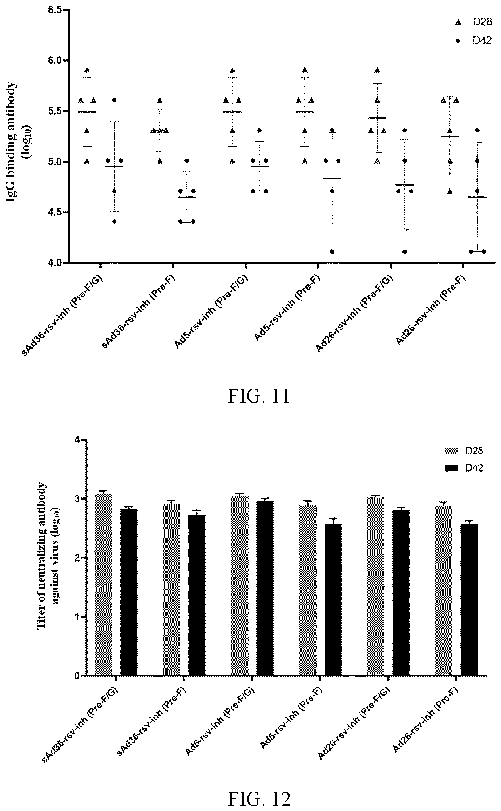

FIG. 11 illustrates the titer of IgG binding antibody 28 days and 42 days after immunization with different immunogen transmucosal formulations; and
FIG. 12 illustrates the titer of neutralizing antibody against RSV A2 virus 28 days and 42 days after immunization with different immunogen transmucosal formulations.

### DETAILED DESCRIPTION

The technical solutions of the present disclosure will be clearly and completely described below with reference to the drawings of the present disclosure, and apparently, the described examples are only exemplary embodiments of the present disclosure instead of all embodiments of the present invention. Based on the examples of the present disclosure, all other examples obtained by those of ordinary skill in the art without creative work shall fall within the protection scope of the present disclosure.

The recombinant adenovirus vectors of the present disclosure, Ad5, Ad26, or sAd36, lack at least one essential gene function in the E1 region of the adenovirus genome required by virus replication. In certain examples, the adenovirus vector of the present disclosure lacks at least a portion of the non-essential E3 region. In certain examples, the vector lacks at least one essential gene function in the E1 region and at least a portion of the non-essential E3 region. The adenovirus vector may be "multi-deficient", which means that two or more regions of the adenovirus genome of the adenovirus vector each lack one or more essential gene functions. The aforementioned E1-deficient or E1/E3-deficient adenovirus vector may additionally lack at least one essential gene in the E4 region and/or at least one essential gene in the E2 region.

In certain examples, the adenovirus is replication-deficient due to, e.g., a deletion in the E1 region of the genome. As known to those of ordinary skill, in case of the deletion of an essential region of the adenovirus genome, the functions encoded by such regions must be provided in trans, preferably by a producer cell. That is, when part or all of the E1, E2, and/or E4 regions are deleted in the adenovirus, such regions must be present in the producer cell, e.g., integrated in its genome or in the form of a so-called helper adenovirus or helper plasmid. The adenovirus may also have deletions in the E3 region that is not essential for replication, and thus do not need to be complemented with such deletions.

The producer cell (sometimes also referred to in the art and herein as a 'packaging cell', 'complementing cell' or 'host cell') that may be used may be any producer cell that can propagate the desired adenovirus. For example, the propagation of the recombinant adenovirus vector is conducted in a producer cell that complements the deficiency in the adenovirus. Such producer cells preferably have in their genome at least the adenovirus E1 sequence and are thus capable of complementing the recombinant adenovirus with a deletion in the E1 region. Any producer cell that is capable of complementing with E1, such as HEK293 cells, PER.C6 cells, 911 cells, IT293SF cells, and the like, can be used.

For Ad5, Ad26, or sAd36 with a deletion in the E1 region, the 3' end of the E1B 55K open reading frame in the adenovirus is preferably retained to further improve the stability of the adenovirus.

The recombinant adenovirus of the present disclosure comprises a nucleic acid encoding an RSV antigen, which may be, for example, cloned into the deleted E1 or E3 region of the adenovirus vector.

The nucleic acid encoding the RSV antigen of the present disclosure is selected from: one or more of the RSV fusion protein F (pre-F), the attachment protein G, and the small hydrophobic (SH) protein. The RSV fusion protein F, attachment protein G, and/or small hydrophobic (SH) protein of RSV may be derived from a native RSV strain or any recombinant RSV strain, preferably from a human RSV strain, such as the A2, Long, or B strain.

In other examples, the nucleic acid encoding the RSV antigen may be based on more than one of the amino acid sequences of the RSV F (pre-F) protein, the attachment protein G, and/or the small hydrophobic (SH) protein.

In some certain examples, the recombinant adenovirus comprises a nucleic acid encoding the RSV F (pre-F) protein, and in some certain examples, the recombinant adenovirus comprises a nucleic acid encoding the RSV F (pre-F)/G proteins to further demonstrate the versatility of the transmucosal formulation of the present disclosure among RSV antigens.

The method for preparing the transmucosal formulation comprises: propagating the recombinant adenovirus Ad5, Ad26, or sAd36 in a host cell culture, isolating and purifying the recombinant adenovirus, and formulating the recombinant adenovirus into a pharmaceutically acceptable transmucosal formulation.

The recombinant adenovirus can be prepared and propagated in host cells according to well-known methods, which require cell culture in host cells infected with the adenovirus. The cell culture can be any type, including adherent cell culture (e.g., cells adhere to a culture vessel or microcarrier surface) and suspension culture. Most large-scale suspension cultures are operated in batch or fed-batch processes, as these processes are the easiest to operate and scale up.

The producer cell is cultured to increase the cell and virus counts and/or virus titers. The cell is cultured such that it is capable of metabolizing, growing, dividing, and/or producing the relevant virus of the present disclosure. This can be achieved by methods well known to those of ordinary skill and includes, but is not limited to, providing nutrients to the cell, for example, in an appropriate culture medium. Suitable media are well known to those of ordinary skill, and are generally available in large quantities from commercial sources or may be customized according to standard protocols. The culture can be conducted in a petri dish, a roller bottle, or a bioreactor using a batch system, a fed-batch system, a continuous system, and the like.

In certain examples, the collected adenovirus is further purified. The adenovirus purification can be conducted in several steps, including purification, ultrafiltration, diafiltration, or chromatographic separation, and the purification can be conducted by the filtration step to remove cellular debris and other impurities from the cell lysate. The ultrafiltration is used to concentrate the virus solution. Diafiltration or buffer exchange using an ultrafilter is used as the means to remove and exchange salts, sugars, and analogs thereof. Methods of optimizing conditions for each purification step were known to those of ordinary skill in the art.

The transmucosal formulation of the present disclosure comprises a pharmaceutically acceptable excipient, including but not limited to one or more of the following: a buffer, a protectant, a stabilizer, a surfactant, an osmotic pressure regulator, an adjuvant, a preservative, an inactivator, and a human albumin, preferably, human serum albumin; the buffer includes, but is not limited to, one or more of HEPES, HIS, TRIS, PB, succinate, and citrate; the protectant includes, but is not limited to, one or more of gelatin, ethanol, ethylenediamine tetraacetate (EDTA), disodium edtate (EDTA-2Na), and magnesium chloride; the stabilizer includes, but is not limited to, one or more of sucrose, mannitol, fucose, and maltose; the surfactant includes, but is not limited to, one or more of Tween, Span, and glycerol; the osmotic pressure regulator includes, but is not limited to, sodium chloride and absence; preferably, the excipient component includes mannitol, sucrose, sodium chloride, magnesium chloride, HEPES, polysorbate 80, and glycerol, preferably, 10-200 mg/mL of mannitol, 0.5-30 mg/mL of sodium chloride, 0.05-10 mg/mL of HEPES, 0.01-10 mg/mL of polysorbate 80, 0.1-5 mg/mL of glycerol, 0.1-5 mg/mL of magnesium chloride, and 1-80 mg/mL of sucrose, and more preferably, 10-80 mg/mL of mannitol, 1-15 mg/mL of sodium chloride, 0.1-5 mg/mL of HEPES, 0.1-5 mg/mL of polysorbate 80, 0.5-5 mg/mL of glycerol, 0.2-5 mg/mL of magnesium chloride, and 5-40 mg/mL of sucrose.

In certain examples, the present disclosure provides a kit for preventing and/or treating respiratory syncytial virus infection, comprising the transmucosal formulation of the present disclosure and a delivery device.

The delivery device may be an inhalation device, e.g., an atomizer or an aerosol generator.

In certain examples, the present disclosure provides use of a transmucosal formulation for inducing an immune response in a mammal, comprising: administering a sufficient amount of the transmucosal formulation to the subject to induce a primary immune response.

In certain examples, the present disclosure provides use of a transmucosal formulation for inducing a booster immune response in a mammal, comprising: administering a sufficient amount of the transmucosal formulation to the subject that has developed a primary immune response against the immunogen of the same species before the use of the transmucosal formulation of the present disclosure.

The subject as used herein is preferably a mammal, e.g., a rodent, e.g., a mouse, a cotton rat, or a non-human primate, or a human. The subject is preferably a human subject. The subject may be any age, e.g., from about 1 month old to 100 years old, e.g., from about 2 months old to about 80 years old, e.g., from about 1 month old to about 3 years old, from about 3 years old to about 50 years old, from about 50 years old to about 75 years old, and the like.

In certain examples, the present disclosure provides a method for treating and/or preventing respiratory syncytial virus infection, comprising: administering to a patient or vaccinee the transmucosal formulation at an amount of 1 × 10⁷ IFU, preferably 1 × 10⁷-6 × 10⁹ IFU, preferably 1 × 10⁷-1.3 × 10⁹ IFU.

In certain examples, the present disclosure provides a method for treating and/or preventing respiratory syncytial virus infection, wherein at least one dose of the transmucosal formulation is administered.

In certain examples, the present disclosure provides a method for treating and/or preventing respiratory syncytial virus infection, wherein the transmucosal formulation may be administered in combination with an immunological composition of a recombinant adenovirus of an identical or different serotype, or may also be administered in combination with a composition of a protein expressed by an RSV antigenic nucleic acid.

In certain examples, the present disclosure provides a method for treating and/or preventing respiratory syncytial virus infection, wherein the transmucosal formulation may be administered in combination with an additional therapeutic agent; the additional therapeutic agent may or may not exert a synergistic effect with the transmucosal formulation of the present disclosure, and the two may be administered through identical or different routes and may be administered at substantially identical or different periods.

The additional therapeutic agent may be selected from, but is not limited to, a therapeutic agent against RSV infection, such as a standard treatment against lower respiratory tract RSV infection during hospitalization, a bronchodilator, and an antibiotic, including (but not limited to) epinephrine, an anticholinergic, an antipyretic, and/or a nonsteroid anti-inflammatory drug.

The bronchodilator includes two main categories, sympathomimetics and anticholinergics, including short-acting and long-acting β2 mimetics. The short-acting mimetics include, but are not limited to: salbutamol, terbutaline, fenoterol, pirbuterol, and tulobuterol. The long-acting β2 mimetics include, but are not limited to, formoterol and salmeterol. They may also be used as bases or as pharmaceutically acceptable salts. The anticholinergics include, but are not limited to, ipratropium bromide, oxitropium bromide, and tiotropium bromide.

Other bronchodilators useful in the method of the present disclosure include, but are not limited to: Accu Hale, salbutamol, bitolterol, ephedrine, epinephrine, isoproterenol, metaproterenol, pirbuterol, acetyl epinephrine, ritodrine, terbutaline, levo, methamphetamine, cocaine, theophylline, caffeine, theobromine, THC, and MDPV.

The inventors of the present disclosure have surprisingly found that the transmucosal formulations of recombinant adenovirus sAd36, Ad26, or Ad5 comprising a nucleotide sequence encoding an RSV antigen were demonstrated to be extremely effective anti-RSV compositions in a mouse model. In order to further verify the advantages of the adenovirus vectors of the present disclosure in mucosal immunity, recombinant adenoviruses sAd37, Ad48, and Ad35 containing nucleotide sequences encoding RSV antigens were constructed and formulated into transmucosal formulations by the same method. The reference vectors were selected from human adenoviruses and simian adenoviruses, so as to more comprehensively compare with the advantages of the vectors in the present disclosure.

In order to further verify the advantages of the transmucosal formulation of the present disclosure, recombinant adenoviruses sAd36, Ad26, and Ad5 containing nucleotide sequences encoding RSV antigens were administered via different routes such as oral aerosol inhalation, nasal aerosol inhalation, nasal drop, and oral dry powder inhalation, so as to more comprehensively compare with the advantages of the transmucosal formulation and determine the most advantageous transmucosal administration routes.

In order to further verify the advantages of the transmucosal formulation of the present disclosure, recombinant adenoviruses sAd36, Ad26, and Ad5 containing nucleotide sequences encoding RSV antigens were administered by oral aerosol inhalation at different doses, so as to further determine the most advantageous dose range.

The technical solutions of the present disclosure will be clearly and completely described below with reference to the examples of the present disclosure, and apparently, the described examples are only exemplary embodiments of the present disclosure instead of all embodiments of the present invention. Based on the examples of the present disclosure, all other examples obtained by those of ordinary skill in the art without creative efforts shall fall within the claimed scope of the present disclosure.

The following examples relate to a model amino acid sequence of the RSV F (pre-F) protein in the prefusion conformation as set forth in SEQ ID NO: 1; the model amino acid sequence of the RSV pre-F/G protein is set forth in SEQ ID NO: 4.

### Example 1: Production and expression of adenovirus vectors sAd36-ΔE1,3, Ad26-ΔE1,3, Ad5-ΔE1,3, sAd37-ΔE1,3, Ad35-ΔE1,3, and Ad48-ΔE1,3

In the adenovirus vector based on simian adenovirus sAd36 (GENBANK No.: FJ025917.1), E1 and E3 genes were deleted;

In the adenovirus vector based on simian adenovirus sAd37 (GENBANK No.: FJ025921), E1 and E3 genes were deleted;

In the adenovirus vector based on human adenovirus 26 (GENBANK No.: EF153474.1), E1 and E3 genes were deleted;

In the adenovirus vector based on human adenovirus 5 (GENBANK No.: AC_000008.1), E1 and E3 genes were deleted;

In the adenovirus vector based on human adenovirus 35 (GENBANK No.: AC_000019.1), E1 and E3 genes were deleted;

In the adenovirus vector based on human adenovirus 48 (GENBANK No.: EF153473.1), E1 and E3 genes were deleted

### Example 2: Cell adaptation test of recombinant viruses sAd36-rsv-F, Ad26-rsv-F, and Ad5-rsv-F

sAd36-rsv-F and Ad26-rsv-F were constructed by reverse genetics. Elements such as ori and resistance genes that are not present in a virus genome were removed by digestion with MssI enzyme. The plasmid was linearized and purified by using a commercial purification and recovery kit to remove enzymes and other reaction components. 1-2 µg of the purified linearized plasmids was used to transfect HEK293SF-3F6 cells in a 6-well plate by using a lipo2000 kit. The cells were transferred to a T25 bottle for culture the next day, so as to remove lipo2000 reagents harmful to the cells and provide the cells with a larger growth space. After 7 days, plaques due to viral infection were observed, which were formed due to cell lysis. sAd37-rsv-F, Ad35-rsv-F, and Ad48-rsv-F were constructed in the same manner.

For the construction of Ad5-rsv-F, an optimized RSV F protein gene was amplified by PCR. The recovered product was digested, and the digested fragments were recovered. Meanwhile, the plasmid pDC316 vector was digested with the same endonuclease, and the recombinant pDC316 vector was recovered. The vector and pBHGloxΔE1,3 Cre were co-transfected into HEK293SF-3F6 cells for virus rescue. After 10-20 days, plaques due to viral infection were observed, which were formed due to cell lysis.

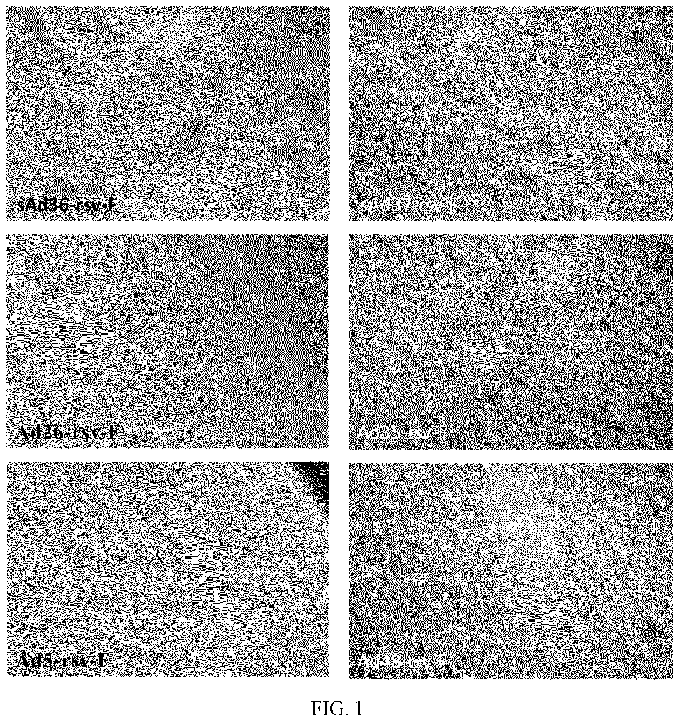

The results, as shown in FIG. 1, show that the recombinant viruses sAd36-rsv-F, Ad26-rsv-F, Ad5-rsv-F, sAd37-rsv-F, Ad35-rsv-F, and Ad48-rsv-F exhibited good adaptation in cells and can meet the requirement of massive production.

Production, infection, and subculture of adenoviruses sAd36-rsv-F, Ad26-rsv-F, and Ad5-rsv-F: Plaques due to viral infection were observed, which were formed due to cell lysis. The cells were cultured for 3-5 days before the viruses were collected. The cells were separated by pipetting, transferred into a 15 mL centrifuge tube, shaken on a shaker to mix uniformly, and aliquoted at 1 mL/vial to give P-2 generation viruses sAd36-rsv-F, Ad26-rsv-F, Ad5-rsv-F, sAd37-rsv-F, Ad35-rsv-F, and Ad48-rsv-F. The virus was then inoculated into a HEK293 cell suspension culture for expansion. P-2 generation viruses were inoculated into 30 mL of the HEK293 cell suspension culture to give P-1 generation viruses, the P-1 generation viruses were inoculated into 500 mL of HEK293S cell suspension culture to give P0 generation viruses. 500 mL of the virus was harvested and purified to prepare virus formulations for animal immunization.

### Example 3: Cell adaptation test of recombinant viruses sAd36-rsv-F/G, Ad26-rsv-F/G, and Ad5-rsv-F/G

sAd36-rsv-F/G and Ad26-rsv-F/G were constructed by reverse genetics. Elements such as ori and resistance genes that are not present in a virus genome were removed by digestion with MssI enzyme. The plasmid was linearized and purified by using a commercial purification and recovery kit to remove enzymes and other reaction components. 1-2 µg of the purified linearized plasmids was used to transfect HEK293SF-3F6 cells in a 6-well plate by using a lipo2000 kit.

The cells were transferred to a T25 bottle for culture the next day, so as to remove lipo2000 reagents harmful to the cells and provide the cells with a larger growth space. After 7 days, blanks due to viral infection were observed, which were formed due to cell lysis.

For the construction of Ad5-rsv-F, an optimized RSV F/G protein gene was amplified by PCR. The recovered product was digested, and the digested fragments were recovered. Meanwhile, the plasmid pDC316 vector was digested with the same endonuclease, and the recombinant pDC316 vector was recovered. The vector and pBHGloxΔE1,3 Cre were co-transfected into HEK293SF-3F6 cells for virus rescue. After 10-20 days, plaques due to viral infection were observed, which were formed due to cell lysis.

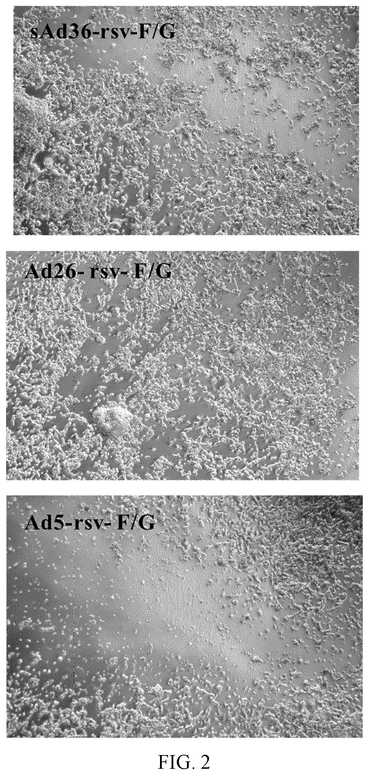

The results, as shown in FIG. 2, show that the recombinant adenoviruses sAd36-rsv-F/G, Ad26-rsv-F/G, and Ad5-rsv-F/G exhibited good adaptation in cells and can meet the requirement of massive production.

The production, infection, and subculture procedures of recombinant adenoviruses sAd36-rsv-F/G, Ad26-rsv-F/G, and Ad5-rsv-F/G are the same as those in Example 2.

### Example 4: Preparation of transmucosal formulation and injection of sAd36-rsv-F, Ad26-rsv-F, Ad5-rsv-F, sAd37-rsv-F, Ad35-rsv-F, and Ad48-rsv-F

Mannitol, sucrose, sodium chloride, magnesium chloride, polysorbate 80, glycerol, and HEPES (hydroxyethyl piperazine ethanesulfonic acid) were added into the stock solutions of viruses sAd36-rsv-F, Ad26-rsv-F, Ad5-rsv-F, sAd37-rsv-F, Ad35-rsv-F, Ad48-rsv-F, sAd36-rsv-F/G, Ad26-rsv-F/G, and Ad5-rsv-F/G to prepare injections.

HEPES buffer, sucrose, isotonic saline, and polysorbate 80 were added into the stock solutions of viruses sAd36-rsv-F, Ad26-rsv-F, Ad5-rsv-F, sAd37-rsv-F, Ad35-rsv-F, Ad48-rsv-F, sAd36-rsv-F/G, Ad26-rsv-F/G, and Ad5-rsv-F/G to prepare oral aerosol inhalation formulations for transmucosal administration. The obtained transmucosal formulation was further prepared into inhalant dry powder particles, i.e., the dry oral powder inhalant formulation, by low-temperature spray drying.

PBS buffer, mannitol, polysorbate 80, and glycerol were added into the stock solutions of viruses sAd36-rsv-F, Ad26-rsv-F, Ad5-rsv-F, sAd37-rsv-F, Ad35-rsv-F, Ad48-rsv-F, sAd36-rsv-F/G, Ad26-rsv-F/G, and Ad5-rsv-F/G to prepare oral aerosol inhalation formulations for transmucosal administration.

Sodium chloride, trehalose, sucrose, glycine, arginine, sodium glutamate, histidine, urea, dextran 40, and human serum albumin were added into the stock solutions of viruses sAd36-rsv-F, Ad26-rsv-F, Ad5-rsv-F, sAd37-rsv-F, Ad35-rsv-F, Ad48-rsv-F, sAd36-rsv-F/G, Ad26-rsv-F/G, and Ad5-rsv-F/G to prepare nasal drop formulations.

### Example 5: Evaluation of humoral immune response of transmucosal formulations and injections

Animal study: BALB/c mice were quarantined according to the Standard Operation Procedures for Quarantine of Experimental Animals (SOP-QCM-050), and 78 qualified mice were selected and randomized into 13 groups. The injection and the transmucosal formulation prepared in Example 4 were respectively used for the experiment according to the administration route and the dose shown in the following Table.

Immunization method: Groups 1-6 received the formulation through oral aerosol inhalation. Immunization procedures: The mice were anesthetized with isoflurane; after at least 20 minutes, the mice were fixed on an operating platform at an angle of about 45° to the benchtop, and the transmucosal formulation prepared above was administered through the mouth into the trachea via an aerosol inhalation device; the mouse head was kept in a higher position for 10-20 s before the mouse was returned to the cage. The mice were kept warm by using an infrared lamp until the mice were awake. Groups 7-12 received the injection intramuscularly in the hind leg.

| **Group number** | **Virus** | **Route of administration** | **Number of immunizations** | **Dose/mouse** |
|---|---|---|---|---|
| Group 1 | sAd36-rsv-F | Intramuscular injection in hind leg | 6 mice | 5×10⁶ IFU |
| Group 2 | Ad5-rsv-F | | 6 mice | 5×10⁶ IFU |
| Group 3 | Ad26-rsv-F | | 6 mice | 5×10⁶ IFU |
| Group 4 | sAd37-rsv-F | | 6 mice | 5×10⁶ IFU |
| Group 5 | Ad35-rsv-F | | 6 mice | 5×10⁶ IFU |
| Group 6 | Ad48-rsv-F | | 6 mice | 5×10⁶ IFU |
| Group 7 | sAd36-rsv-F | Aerosol inhalation | 6 mice | 1×10⁶ IFU |
| Group 8 | Ad5-rsv-F | | 6 mice | 1×10⁶ IFU |
| Group 9 | Ad26-rsv-F | | 6 mice | 1×10⁶ IFU |
| Group 10 | sAd37-rsv-F | | 6 mice | 1×10⁶ IFU |
| Group 11 | Ad35-rsv-F | | 6 mice | 1×10⁶ IFU |
| Group 12 | Ad48-rsv-F | | 6 mice | 1×10⁶ IFU |
| Group 13 | Negative control - sodium chloride injection | Intramuscular injection in hind leg | 6 mice | 100 µL |

Methodology: Evaluation of titer of IgG binding antibody against pre-F: The Cutoff value was calculated according to the serum absorbance value of the normal saline control group, and the serum antibody titers in the vaccination groups were calculated. The maximum dilution factor higher than the cutoff value is the titer of the antibody. Cutoff value = mean serum absorbance value of mice in negative control group × 2.1.

Detection of neutralizing antibodies against RSV A2 virus

Humoral immunity results: On day 28, blood samples were collected to detect the titer of IgG binding antibody against pre-F and the titer of neutralizing antibody against RSV A2 virus.

**Table 1. Serum titer of IgG binding antibody against pre-F in mice 28d after the immunization with transmucosal formulations or injections of sAd36-rsv-F/Ad26-rsv-F/Ad5-rsv-F/sAd37-rsv-F/Ad35-rsv-F/Ad48-rsv-F**

| **Group** | **Titer of IgG binding antibody against pre-F** | | | | | |
|---|---|---|---|---|---|---|
| | **Mouse 1** | **Mouse 2** | **Mouse 3** | **Mouse 4** | **Mouse 5** | **Mouse 6** |
| sAd36-rsv-i.m. | 3.806 | 4.107 | 4.107 | 4.408 | 4.107 | 3.204 |
| Ad5-rsv-i.m. | 4.709 | 4.408 | 3.806 | 4.107 | 3.806 | 3.505 |
| Ad26-rsv-i.m. | 5.612 | 5.01 | 5.311 | 5.612 | 5.01 | 5.01 |
| sAd37-rsv-i.m. | 3.505 | 4.107 | 3.505 | 3.806 | 5.408 | 4.408 |
| Ad35-rsv-i.m. | 5.01 | 5.311 | 5.01 | 5.01 | 5.311 | 5.01 |
| Ad48-rsv-i.m. | 5.01 | 4.709 | 5.01 | 4.709 | 5.101 | 5.612 |
| sAd36-rsv-I.H. | 5.612 | 5.612 | 5.612 | 5.612 | 5.913 | 5.612 |
| Ad5-rsv-I.H. | 6.21 | 6.515 | 5.311 | 5.612 | 5.612 | 5.913 |
| Ad26-rsv-I.H.. | 5.612 | 5.913 | 5.612 | 6.214 | 5.612 | 5.311 |
| sAd37-rsv-I.H.. | 5.408 | 5.311 | 5.01 | 5.01 | 5.311 | 5.01 |
| Ad35-rsv-I.H. | 5.01 | 4.709 | 4.709 | 4.709 | 4.408 | 5.01 |
| Ad48-rsv-I.H. | 4.408 | 4.709 | 5.01 | 5.01 | 4.709 | 4.709 |
| Control group | 0 | 0 | 0 | 0 | 0 | 0 |

| | | | | | | |
|---|---|---|---|---|---|---|
| *sAd36-rsv-F, Ad26-rsv-F, Ad5- rsv-F, sAd37- rsv-F, Ad35- rsv-F, and Ad48-rsv-F transmucosal formulations and injections were given at the same dose, blood samples were collected to detect the IgG binding antibody titer 28 days after the immunization, wherein i.m. denotes intramuscular injection, while i.n. denotes transmucosal formulation for oral aerosol inhalation. The data of groups 1-6 show that among groups receiving the injection, Ad26-rsv-F, Ad35-rsv-F, and Ad48-rsv-F induced higher titers of IgG binding antibodies; the data of groups 7-12 show that among groups receiving the transmucosal formulation through aerosol inhalation, sAd36-rsv-F, Ad26-rsv-F, and Ad5-rsv-F induced higher titers of IgG binding antibodies. | | | | | | |

**Table 2. Serum titer of neutralizing antibody against RSV A2 virus in mice 28d after the immunization with transmucosal formulations or injections of sAd36-rsv-F/Ad26-rsv-F/Ad5-rsv-F/sAd37-rsv-F/Ad35-rsv-F/Ad48-rsv-F**

| **Group** | **Titer of neutralizing antibody against virus** | | | | | |
|---|---|---|---|---|---|---|
| | **Mouse 1** | **Mouse 2** | **Mouse 3** | **Mouse 4** | **Mouse 5** | **Mouse 6** |
| sAd36-rsv-i.m. | 82 | 74 | 53 | 91 | 68 | 84 |
| Ad5-rsv-i.m. | 62 | 54 | 41 | 59 | 66 | 50 |
| Ad26-rsv-i.m. | 98 | 112 | 116 | 124 | 136 | 154 |
| sAd37-rsv-i.m. | 89 | 92 | 73 | 80 | 76 | 80 |
| Ad35-rsv-i.m. | 201 | 210 | 180 | 159 | 195 | 144 |
| Ad48-rsv-i.m. | 172 | 184 | 166 | 178 | 158 | 146 |
| sAd36-rsv-I.H. | 522 | 480 | 570 | 390 | 528 | 522 |
| Ad5-rsv-I.H. | 609 | 567 | 507 | 675 | 780 | 525 |
| Ad26-rsv-I.H. | 528 | 654 | 567 | 690 | 582 | 606 |
| sAd37-rsv-I.H. | 282 | 336 | 249 | 228 | 447 | 216 |
| Ad35-rsv-I.H. | 132 | 160 | 144 | 96 | 124 | 242 |
| Ad48-rsv-I.H. | 207 | 231 | 146 | 174 | 219 | 101 |
| Negative control | 0 | 0 | 0 | 0 | 0 | 0 |

| | | | | | | |
|---|---|---|---|---|---|---|
| *Groups 1 and 7, 2 and 8, 3 and 9, 4 and 10, 5 and 11, 6 and 12 received the same recombinant adenovirus serotype. Groups 1-6 received the transmucosal formulation through oral aerosol inhalation, while groups 7-12 received the injection intramuscularly in the hind leg. | | | | | | |

According to the result, the neutralizing antibody was better induced by the transmucosal formulations prepared from recombinant adenovirus sAd36-rsv-F, Ad26-rsv-F, and Ad5-rsv-F than the injections, suggesting that the transmucosal formulation can better induce the humoral immune response in the body.

For different bacterial or viral antigens and different administration routes, vectors of different serotypes should be screened to match the virus vector vaccine. Specifically, for RSV, according to data of recombinant adenovirus vectors of different serotypes, the transmucosal formulations of sAd36-rsv-F, Ad26-rsv-F, and Ad5-rsv-F exhibited higher advantages in elevating neutralizing antibody titer as compared with the corresponding injections, indicating that, for RSV, adenoviruses of serotypes sAd36, Ad26, and Ad5 are more advantageous when prepared into transmucosal formulations than other serotypes.

### Example 6: Evaluation of cellular immunity and mucosal immunity of transmucosal formulations and injections

Animal study: BALB/c mice were quarantined according to the Standard Operation Procedures for Quarantine of Experimental Animals (SOP-QCM-050), and 96 qualified mice were selected and randomized into 16 groups.

The transmucosal formulation and the transmucosal formulation dry powder particles prepared in Example 4 were administered for immunization according to the following administration routes and doses.

| **Group** | **Virus** | **Route of administration** | | **Number of immunizations** | **Dose/mouse** |
|---|---|---|---|---|---|
| Group 1 | sAd36-rsv-F | Oral aerosol inhalation | IH-oral | 6 mice | 5×10⁷ IFU |
| Group 2 | | Nasal aerosol inhalation | IH-nose | 6 mice | 5×10⁷ IFU |
| Group 3 | | Nasal drop | i.n. | 6 mice | 5×10⁷ IFU |
| Group 4 | | Oral dry powder inhalation | DPI | 6 mice | 5×10⁷ IFU |
| Group 5 | | Intramuscular injection | i.m. | 6 mice | 1×10⁸ IFU |
| Group 6 | Ad5-rsv-F | Oral aerosol inhalation | IH-oral | 6 mice | 5×10⁷ IFU |
| Group 7 | | Nasal aerosol inhalation | IH-nose | 6 mice | 5×10⁷ IFU |
| Group 8 | | Nasal drop | i.n. | 6 mice | 5×10⁷ IFU |
| Group 9 | | Oral dry powder inhalation | DPI | 6 mice | 5×10⁷ IFU |
| Group 10 | | Intramuscular injection | i.m. | 6 mice | 1×10⁸ IFU |
| Group 11 | Ad26-rsv-F | Oral aerosol inhalation | IH-oral | 6 mice | 5×10⁷ IFU |
| Group 12 | | Nasal aerosol inhalation | IH-nose | 6 mice | 5×10⁷ IFU |
| Group 13 | | Nasal drop | i.n. | 6 mice | 5×10⁷ IFU |
| Group 14 | | Oral dry powder inhalation | DPI | 6 mice | 5×10⁷ IFU |
| Group 15 | | Intramuscular injection | i.m. | 6 mice | 1×10⁸ IFU |
| Group 16 | Negative control - sodium chloride injection | Intramuscular injection in hind leg | NS | 6 mice | 100 µL |

Groups 1, 6, and 11 received the oral aerosol inhalation. Immunization procedures: The mice were anesthetized with isoflurane; after at least 20 minutes, the mice were fixed on an operating platform at an angle of about 45° to the benchtop, and the transmucosal formulation prepared above was administered through the mouth into the trachea via an aerosol inhalation device; the mouse head was kept in a higher position for 10-20 s before the mouse was returned to the cage. The mice were kept warm by using an infrared lamp until the mice were awake.

Groups 2, 7, and 12 received the nasal aerosol inhalation. Immunization procedures: The mice were anesthetized with isoflurane; after at least 20 minutes, the mice were fixed on an operating platform at an angle of about 45° to the benchtop, and the transmucosal formulation prepared in Example 4 was administered through the nose via an aerosol inhalation device. The mice were returned to the cage and kept warm by using an infrared lamp until the mice were awake. Groups 3, 8, and 13 received the nasal drop. Immunization procedures: The mice were anesthetized with isoflurane; after 5-10 minutes, the mice were fixed on an operating platform at an angle of about 45° to the benchtop, and the transmucosal formulation prepared in Example 4 was administered through nasal drop; the mouse head was kept in a higher position for 10-20 s before the mouse was returned to the cage. The mice were kept warm by using an infrared lamp until the mice were awake.

Groups 4, 9, and 14 received the oral dry powder inhalation. Immunization procedures: The mice were anesthetized with isoflurane; after at least 20 minutes, the mice were fixed on an operating platform at an angle of about 45° to the benchtop, and the transmucosal formulation dry powder prepared in Example 4 was administered through the mouse into the trachea via an aerosol inhalation device; the mouse head was kept in a higher position for 10-20 s before the mouse was returned to the cage. The mice were kept warm by using an infrared lamp until the mice were awake.

Groups 5, 10, and 15 received the injection intramuscularly in the hind leg with the injection prepared in Example 4.

Methodology: the ICS method.

Results of cellular immunity study:
The mice were sacrificed on day 28, and the splenocytes were collected for ICS assay to determine the TNF-α, IFN-y, and IL-2 response levels of CD4+ and CD8+ T cells, so as to evaluate the cellular immune response induced by the transmucosal formulations and injections; the cellular immune responses induced by different administration routes of the transmucosal formulation were also evaluated.

**Table 3. TNF-α, IFN-γ, and IL-2 response levels of SPL CD8+ T cell 28d after mice were immunized with a transmucosal formulation or injection prepared from sAd36-rsv-F/Ad26-rsv-F/Ad5-rsv-F.**

| **Group** | **TNFα+** | | | | | | **IFNγ+** | | | | | | **IL2+** | | | | | |
|---|---|---|---|---|---|---|---|---|---|---|---|---|---|---|---|---|---|---|
| | Mouse 1 | Mouse 2 | Mouse 3 | Mouse 4 | Mouse 5 | Mouse 6 | Mouse 1 | Mouse 2 | Mouse 3 | Mouse 4 | Mouse 5 | Mouse 6 | Mouse 1 | Mouse 2 | Mouse 3 | Mouse 4 | Mouse 5 | Mouse 6 |
| sAd36-rsv-IH-nose | 3.75 | 6.35 | 5.22 | 9.76 | 5.81 | 7.12 | 7.63 | 6.08 | 7.29 | 11.07 | 9.72 | 7.95 | 1.78 | 2.14 | 2.03 | 0.99 | 1.59 | 1.3 |
| sAd36-rsv-IH-oral | 11.45 | 13.8 | 10.68 | 8.49 | 12.17 | 7.54 | 10.94 | 13.95 | 12.95 | 16.3 | 7.85 | 18.88 | 1.68 | 2.33 | 2.17 | 1.49 | 1.57 | 2.08 |
| sAd36-rsv-i.n. | 2.7 | 6 | 5.72 | 3.57 | 5.3 | 3.64 | 4.46 | 8.61 | 8.66 | 5.02 | 8.53 | 7.17 | 1.51 | 1.01 | 1.22 | 0.42 | 0.72 | 0.88 |
| sAd36-rsv-DPI | 3.08 | 3.12 | 3.4 | 4.6 | 3.88 | 4.23 | 5.17 | 3.29 | 2.53 | 2.07 | 5.12 | 3.89 | 0.18 | 1.88 | 0.15 | 0.08 | 2.23 | 1.77 |
| sAd36-rsv-i.m. | 2.14 | 1.76 | 1.03 | 1.67 | 3.44 | 2.74 | 1.31 | 4.24 | 5.04 | 2.4 | 2.77 | 1.03 | 0.18 | 0.93 | 1.05 | 0.81 | 0.67 | 0.89 |
| Ad5-rsv-IH-nose | 6.75 | 9.69 | 12.7 | 9.47 | 4.85 | 5.65 | 4.42 | 15.4 | 8.89 | 10.67 | 5.6 | 7.07 | 1.59 | 2.33 | 1.78 | 2.11 | 2.17 | 1.27 |
| Ad5-rsv-IH-oral | 14.45 | 11.83 | 14.81 | 12.03 | 4.26 | 9.13 | 19.37 | 17.56 | 20.12 | 17.44 | 8.67 | 13.28 | 2.75 | 2.95 | 2.05 | 1.99 | 2.72 | 2.85 |
| Ad5-rsv-i.n. | 5.43 | 3.25 | 5.87 | 6.24 | 5.09 | 2.16 | 6.47 | 6.32 | 5.43 | 4.19 | 3.54 | 2.18 | 1.07 | 1.54 | 1.98 | 0.96 | 1.15 | 2.86 |
| Ad5-rsv-DPI | 2.48 | 1.88 | 3.34 | 5.33 | 1.45 | 1.5 | 5.68 | 1.69 | 1.48 | 2.44 | 3.78 | 4.17 | 1.76 | 0.55 | 1.09 | 1.58 | 1.89 | 1.97 |
| Ad5-rsv-i.m. | 2.36 | 0.33 | 2.89 | 3.31 | 1.38 | 1.62 | 1.36 | 2.87 | 1.61 | 1.94 | 2.07 | 4.99 | 1.94 | 1.88 | 2.24 | 1.75 | 1.67 | 0.01 |
| Ad26-rsv-IH-nose | 6.47 | 13.5 | 5.12 | 8.73 | 10.92 | 6.71 | 16.29 | 3.01 | 10.24 | 7.9 | 9.63 | 8.2 | 1.08 | 2.18 | 1.66 | 2.48 | 1.48 | 1.17 |
| Ad26-rsv-IH-oral | 13.65 | 12.71 | 10.49 | 11.31 | 8.76 | 11.77 | 13.75 | 17.43 | 15.6 | 12.58 | 9.79 | 14.44 | 2.17 | 2.22 | 1.78 | 2.79 | 0.98 | 1.05 |
| Ad26-rsv-i.n. | 2.08 | 3.31 | 5.8 | 7.05 | 3.23 | 4.71 | 7.56 | 5.34 | 5.07 | 4.28 | 4.65 | 3.18 | 0.79 | 0.77 | 0.79 | 1.13 | 1.06 | 0.93 |
| Ad26-rsv-DPI | 1.49 | 2.25 | 1.54 | 2.77 | 4.11 | 3.34 | 5.92 | 6.67 | 5.2 | 2.11 | 4.38 | 3.05 | 0.73 | 1.22 | 0.2 | 0.23 | 0.16 | 1.21 |
| Ad26-rsv-i.m. | 2.65 | 3.37 | 1.98 | 2.08 | 4.53 | 1.27 | 1.51 | 2.85 | 2.46 | 1.03 | 5.59 | 2.51 | 0.41 | 0.1 | 0.24 | 0.38 | 0.56 | 0.75 |
| NS | 0.35 | 0.01 | 0.01 | 0.19 | 0.01 | 0.01 | 0.01 | 0.01 | 0.01 | 0.01 | 0.01 | 0.01 | 0.01 | 0.01 | 0.01 | 0.01 | 0.07 | 0 |

**Table 4. TNF-α, IFN-γ, and IL-2 response levels of SPL CD4+ T cell 28d after mice were immunized with a transmucosal formulation or injection prepared from sAd36-rsv-F/Ad26-rsv-F/Ad5-rsv-F**

| **Group** | **TNFα+** | | | | | | **IFNγ+** | | | | | | **IL2+** | | | | | |
|---|---|---|---|---|---|---|---|---|---|---|---|---|---|---|---|---|---|---|
| | Mouse 1 | Mouse 2 | Mouse 3 | Mouse 4 | Mouse 5 | Mouse 6 | Mouse 1 | Mouse 2 | Mouse 3 | Mouse 4 | Mouse 5 | Mouse 6 | Mouse 1 | Mouse 2 | Mouse 3 | Mouse 4 | Mouse 5 | Mouse 6 |
| sAd36-rsv-IH-nose | 0.79 | 1.59 | 0.85 | 0.89 | 1.51 | 1.39 | 0.87 | 1.82 | 0.91 | 0.57 | 1.54 | 0.66 | 1.48 | 0.55 | 0.89 | 1.05 | 0.3 | 0.74 |
| sAd36-rsv-IH-oral | 1.65 | 1.41 | 1.29 | 1.87 | 1.27 | 1.28 | 1.74 | 1.28 | 1.45 | 1.55 | 0.73 | 1.18 | 1.16 | 1.33 | 1.08 | 1.17 | 1.41 | 1.04 |
| sAd36-rsv-i.n. | 1.43 | 0.88 | 1.22 | 1.19 | 0.47 | 0.62 | 0.88 | 1.54 | 0.58 | 0.27 | 0.93 | 0.58 | 0.59 | 0.74 | 0.68 | 0.35 | 0.43 | 0.48 |
| sAd36-rsv-DPI | 0.76 | 0.89 | 0.66 | 0.3 | 0.49 | 0.49 | 0.78 | 0.61 | 0.57 | 0.8 | 0.64 | 0.48 | 0.3 | 0.32 | 0.46 | 0.45 | 0.48 | 0.44 |
| sAd36-rsv-i.m. | 0.24 | 0.57 | 0.54 | 0.44 | 0.33 | 0.4 | 0.24 | 0.38 | 0.43 | 0.3 | 0.29 | 0.47 | 0.31 | 0.18 | 0.21 | 0.17 | 0.21 | 0.14 |
| Ad5-rsv-IH-nose | 1.52 | 0.87 | 1.73 | 0.89 | 1.58 | 1.95 | 1.65 | 0.61 | 0.39 | 1.27 | 1.17 | 1.29 | 0.48 | 0.31 | 0.72 | 0.32 | 0.98 | 1.16 |
| Ad5-rsv-IH-oral | 1.78 | 1.74 | 1.55 | 1.08 | 1.96 | 1.67 | 1.56 | 1.35 | 1.55 | 1.21 | 1.48 | 1.74 | 1.22 | 0.7 | 0.69 | 0.79 | 0.58 | 0.68 |
| Ad5-rsv-i.n. | 1.61 | 1.11 | 1.28 | 0.87 | 1.67 | 1.55 | 0.33 | 0.53 | 0.7 | 0.72 | 0.65 | 0.5 | 0.25 | 0.3 | 0.39 | 0.65 | 0.45 | 0.34 |
| Ad5-rsv-DPI | 1.39 | 0.42 | 0.38 | 0.72 | 0.67 | 0.22 | 0.44 | 0.42 | 0.55 | 0.57 | 0.4 | 0.49 | 0.57 | 0.43 | 0.33 | 0.28 | 0.31 | 0.49 |
| Ad5-rsv-i.m. | 0.38 | 0.29 | 0.47 | 0.44 | 0.45 | 0.27 | 0.21 | 0.17 | 0.45 | 0.32 | 0.19 | 0.3 | 0.24 | 0.43 | 0.44 | 0.3 | 0.22 | 0.43 |
| Ad26-rsv-IH-nose | 1.18 | 1.54 | 1.48 | 0.39 | 1.76 | 1.52 | 1.22 | 0.98 | 1.18 | 1.28 | 0.85 | 1.08 | 1.08 | 0.65 | 0.54 | 0.87 | 0.43 | 0.73 |
| Ad26-rsv-IH-oral | 1.64 | 1.13 | 1.75 | 1.28 | 1.64 | 1.29 | 1.44 | 1.17 | 1.26 | 1.39 | 1.04 | 1.12 | 1.18 | 1.1 | 0.34 | 0.71 | 0.89 | 0.96 |
| Ad26-rsv-i.n. | 1.16 | 1.23 | 1.24 | 1.09 | 1.07 | 1.2 | 0.41 | 0.37 | 1.44 | 0.83 | 0.55 | 0.91 | 0.88 | 0.7 | 0.72 | 0.53 | 0.44 | 1.06 |
| Ad26-rsv-DPI | 1.47 | 1.08 | 1.12 | 1.09 | 0.99 | 1.34 | 0.33 | 0.49 | 0.4 | 0.52 | 0.33 | 0.57 | 0.48 | 0.28 | 0.32 | 0.41 | 0.41 | 0.25 |
| Ad26-rsv-i.m. | 0.19 | 0.34 | 0.26 | 0.74 | 0.86 | 0.32 | 0.11 | 0.08 | 0.13 | 0.2 | 0.05 | 0.15 | 0.2 | 0.16 | 0.24 | 0.22 | 0.08 | 0.15 |
| NS | 0.05 | 0.08 | 0.01 | 0.1 | 0.01 | 0 | 0.01 | 0.04 | 0.01 | 0.01 | 0.07 | 0 | 0.02 | 0.01 | 0.01 | 0.01 | 0.01 | 0 |

Data analysis of groups 1-5: For recombinant adenovirus sAd36-rsv-F, groups 1-4 received sAd36-rsv-F transmucosal formulations, and group 5 received the sAd36-rsv-F injection. The dose was 5 × 10⁷ IFU for the transmucosal formulation and 1 × 10⁸ IFU for the injection. The dose of the transmucosal formulation was halved. The TNF-α, IFN-y, and IL-2 response levels of splenic CD4+ and CD8+ T cells in group 5 were lower, and the TNF-α, IFN-y, and IL-2 response levels of splenic CD4+ and CD8+ T cells in groups 1-4 were significantly higher than those of group 5. The comparison of groups 1-4 shows that the TNF-α, IFN-y, and IL-2 response levels of CD4+ and CD8+ T cells induced by different administration routes of the transmucosal formulation were different, specifically, oral aerosol inhalation > nasal aerosol inhalation > nasal drop > oral dry powder inhalation. The preferred administration routes of the transmucosal formulation are oral aerosol inhalation or nasal aerosol inhalation.

Data analysis of groups 6-10: For recombinant adenovirus Ad5-rsv-F, groups 6-9 received Ad5-rsv-F transmucosal formulations, and group 10 received the Ad5-rsv-F injection. The dose was 5 × 10⁷ IFU for the transmucosal formulation and 1 × 10⁸ IFU for the injection. The dose of the transmucosal formulation was halved. The TNF-α, IFN-y, and IL-2 response levels of splenic CD4+ and CD8+ T cells in group 10 were lower, and the TNF-α, IFN-y, and IL-2 response levels of splenic CD4+ and CD8+ T cells in groups 6-9 were significantly higher than those of group 10. The comparison of groups 6-9 shows that the TNF-α, IFN-γ, and IL-2 response levels of CD4+ and CD8+ T cells induced by different administration routes of the transmucosal formulation were different, specifically, oral aerosol inhalation > nasal aerosol inhalation > nasal drop > oral dry powder inhalation, suggesting that the preferred administration routes of the transmucosal formulation are oral aerosol inhalation or nasal aerosol inhalation.

Data analysis of groups 11-15: For recombinant adenovirus Ad26-rsv-F, groups 11-14 received Ad26-rsv-F transmucosal formulations, and group 15 received the Ad26-rsv-F injection. The dose was 5 × 10⁷ IFU for the transmucosal formulation and 1 × 10⁸ IFU for the injection. The dose of the transmucosal formulation was halved. The TNF-α, IFN-y, and IL-2 response levels of splenic CD4+ and CD8+ T cells in group 15 were lower, and the TNF-α, IFN-γ, and IL-2 response levels of splenic CD4+ and CD8+ T cells in groups 11-14 were significantly higher than those of group 15. The comparison of groups 11-14 shows that the TNF-α, IFN-y, and IL-2 response levels of CD4+ and CD8+ T cells induced by different administration routes of the transmucosal formulation were different, specifically, oral aerosol inhalation > nasal aerosol inhalation > nasal drop > oral dry powder inhalation. The preferred administration routes of the transmucosal formulation are oral aerosol inhalation or nasal aerosol inhalation.

The data of groups 5, 10, and 15 indicate that the poor clinical efficacy of various RSV vaccines in the prior art may be due to the difficulties in inducing effective cellular immune responses in the body. The data of groups 1-4, 6-9, and 11-14 show that compared with the injection, the transmucosal formulation induced significantly improved cellular immune response in the body even if the administration dose was halved, suggesting that the transmucosal formulation has outstanding advantages in inducing the cellular immune response in the body, and can better induce the cellular immune response and improve the immunity in the body.

Results of mucosal immunity study: BALF cells were collected from mice on day 28 by pulmonary instillation, centrifuged, and subjected to ICS assay to detect the TNF-α, IFN-y, and IL-2 response levels of CD4+ and CD8+ T cells, so as to evaluate mucosal immune response induced by the transmucosal formulation and the injection and different administration routes of the transmucosal formulation.

**Table 5. TNF-α, IFN-γ, and IL-2 response levels of BALF CD8+ T cell 28d after mice were immunized with a transmucosal formulation or injection prepared from sAd36-rsv-F/Ad26-rsv-F/Ad5-rsv-F**

| **Group** | **TNFα+** | | | | | **IFNγ+** | | | | | | | **IL2+** | | | | | |
|---|---|---|---|---|---|---|---|---|---|---|---|---|---|---|---|---|---|---|
| | **Mouse 1** | **Mouse 2** | **Mouse 3** | **Mouse 4** | **Mouse 5** | **Mouse 6** | **Mouse 1** | **Mouse 2** | **Mouse 3** | **Mouse 4** | **Mouse 5** | **Mouse 6** | **Mouse 1** | **Mouse 2** | **Mouse 3** | **Mouse 4** | **Mouse 5** | **Mouse 6** |
| sAd36-rsv-inh-NA | 3530.3 | 4351.9 | 3073.5 | 4383.6 | 5615.5 | 2537.2 | 3645.3 | 4763.9 | 4241.5 | 5579.0 | 4693.2 | 5352.1 | 1745.0 | 1793.5 | 2881.9 | 1505.3 | 1345.3 | 1595.1 |
| sAd36-rsv-inh-OU | 4856.5 | 5841.6 | 6498.7 | 6207.4 | 5867.5 | 4902.0 | 7096.6 | 8345.4 | 6870.3 | 5903.8 | 7268.0 | 7529.4 | 1375.7 | 2075.6 | 1666.4 | 1895.3 | 1336.7 | 2365.0 |
| sAd36-rsv-NA | 1958.7 | 988.5 | 1580.3 | 1718.6 | 2492.8 | 1371.9 | 3576.4 | 4624.7 | 2446.7 | 1604.8 | 4389.5 | 3628.1 | 1264.3 | 673.4 | 467.8 | 864.5 | 938.9 | 1029.5 |
| sAd36-rsv-DPI | 523.4 | 1095.2 | 310.1 | 636.8 | 593.5 | 1198.6 | 1372.5 | 463.8 | 2229.2 | 415.4 | 1375.5 | 1304.3 | 794.7 | 327.5 | 447.7 | 475.9 | 550.0 | 331.8 |
| Ad26-rsv-i.m. | 0 | 0 | 8.3 | 1.7 | 1.7 | 8.3 | 3.3 | 0 | 0 | 6.7 | 0 | 0 | 1.7 | 0 | 0 | 5 | 0 | 0 |
| Ad5-rsv-inh-NA | 860.2 | 3572.4 | 4957.7 | 5456.1 | 5017.7 | 2598.9 | 3577.0 | 4467.6 | 6526.3 | 8515.0 | 7648.2 | 4609.3 | 1207.5 | 1453.7 | 2087.6 | 1769.6 | 1424.5 | 1339.7 |
| Ad5-rsv-inh-OU | 5908.7 | 6756.4 | 4509.2 | 4140.8 | 7154.8 | 7133.1 | 7600.5 | 9123.5 | 6157.9 | 5071.0 | 9440.2 | 9380.2 | 991.8 | 2655.5 | 1268.6 | 1303.6 | 1933.7 | 2198.8 |
| Ad5-rsv-NA | 3734.5 | 1479.5 | 2235.7 | 2561.2 | 3214.2 | 2754.9 | 5428.6 | 4438.7 | 2547.3 | 3189.7 | 4053.7 | 1758.6 | 865.6 | 721.8 | 498.3 | *553.5* | 631.8 | 823.7 |
| Ad5-rsv-DPI | 1548.7 | 1244.6 | 880.7 | 1455.5 | 1135.2 | 1738.9 | 1847.5 | 1743.6 | 1243.0 | 1164.9 | 2185.6 | 2847.4 | 311.7 | 165 | 206.7 | 200.0 | 988.5 | 185.0 |
| Ad5-rsv-i.m. | 1.7 | 0 | 0 | 8.3 | 1.7 | 0 | 5 | 10 | 3.3 | 1.7 | 6.7 | 1.7 | 0 | 0 | 1.7 | 0 | 1.7 | 0 |
| Ad26-rsv-inh-OU | 3414.5 | 5547.2 | 4158.7 | 2839.6 | 5022.5 | 3876.0 | 6538.7 | 4327.5 | 7669.0 | 3328.8 | 5508.1 | 4892.4 | 1865.9 | 1342.1 | 2123.9 | 1543.8 | 1476.4 | 2013.7 |
| Ad26-rsv-inh-NA | 6273.5 | 7028.4 | 3599.8 | 4946.5 | 5146.2 | 6459.3 | 8534.5 | 7856.4 | 7039.6 | 5237.8 | 6415.9 | 5542.3 | 2067.7 | 1894.5 | 2438.8 | **1384.4** | 1638.0 | 1559.4 |
| Ad26-rsv-NA | 2135.4 | 1836.6 | 897.4 | 2530.4 | 1158.6 | 1934.8 | 3382.5 | 1705.4 | 2177.8 | 1312.8 | 3731.4 | 2847.4 | 1365.9 | 1669.8 | 1032.1 | 897.5 | 1998.6 | 1577.4 |
| Ad26-rsv-DPI | 785.4 | 1148.7 | 678.3 | 1557.8 | 1087.4 | 825.7 | 2103.8 | 1178.6 | 2110.4 | 1905.4 | 4718.01 | 1662.0 | 86.7 | 131.7 | 43.3 | 105.0 | 91.7 | 171.7 |
| Ad26-rsv-i.m. | 0 | 5 | 1.7 | 1.7 | 1.7 | 0 | 1.7 | 8.3 | 0 | 1.7 | 0 | 1.7 | 1.7 | 0 | 0 | 0 | 0 | 0 |
| NS | 0 | 0 | 0 | 0 | 0 | 0 | 0 | 0 | 0 | 0 | 0 | 0 | 0 | 0 | 0 | 0 | 0 | 0 |

**Table 6. TNF-α, IFN-γ, and IL-2 response levels of BALF CD4+ T cell 28d after mice were immunized with a transmucosal formulation or injection prepared from sAd36-rsv-F/Ad26-rsv-F/Ad5-rsv-F**

| **Group** | **TNFα+** | | | | | | **IFNy+** | | | | | | **IL2+** | | | | | |
|---|---|---|---|---|---|---|---|---|---|---|---|---|---|---|---|---|---|---|
| | **Mouse 1** | **Mouse 2** | **Mouse 3** | **Mouse 4** | **Mouse 5** | **Mouse 6** | **Mouse 1** | **Mouse 2** | **Mouse 3** | **Mouse 4** | **Mouse 5** | **Mouse 6** | **Mouse 1** | **Mouse 2** | **Mouse 3** | **Mouse 4** | **Mouse 5** | **Mouse 6** |
| sAd36-rsv-inh-NA | 174.7 | 148.1 | 210.7 | 70.6 | 142.3 | 87.1 | 156.8 | 215.7 | 123.8 | 207.5 | 75.4 | 177.4 | 73.3 | 60.0 | 108.3 | 88.6 | 92.3 | 68.3 |
| sAd36-rsv-inh-OU | 136.7 | 199.3 | 151.9 | 286.2 | 91.7 | 143.3 | 252.6 | 154.4 | 271.5 | 212.2 | 161.3 | 273.6 | 75.7 | 56.7 | 98.3 | 110.5 | 135.1 | 141.7 |
| sAd36-rsv-NA | 161.5 | 95.6 | 82.4 | 71.7 | 185.4 | 155.2 | 56.7 | 196.7 | 93.4 | 100.6 | 133.4 | 156.7 | 64.3 | 55.7 | 95.4 | 43.9 | 59.6 | 84.7 |
| sAd36-rsv-DPI | 89.3 | 130.2 | 110.0 | 112.9 | 71.9 | 112.5 | 53.3 | 58.3 | 38.5 | 55.8 | 70.4 | 28.3 | 21.7 | 35.4 | 54.8 | 23.3 | 44.9 | 48.0 |
| Ad26-rsv-i.m. | 5 | 1.7 | 0 | 1.7 | 0 | 0 | 1.7 | 0 | 0 | 0 | 0 | 1.7 | 1.7 | 1.7 | 0 | 0 | 0 | 0 |
| Ad5-rsv-inh-NA | 128.8 | 179.4 | 86.8 | 189.0 | 104.4 | 71.7 | 163.9 | 157.3 | 228.2 | 176.3 | 288.2 | 86.6 | 75.8 | 95.8 | 79.6 | 100.6 | 88.4 | 55.7 |
| Ad5-rsv-inh-OU | 217.7 | 178.4 | 155.9 | 297.0 | 168.5 | 144.2 | 195.0 | 126.7 | 236.7 | 385.1 | 385.1 | 195.0 | 117.5 | 109.6 | 88.3 | 75.3 | 165.8 | 93.3 |
| Ad5-rsv-NA | 38.3 | 28.3 | 21.7 | 40.0 | 41.7 | 15.0 | 41.0 | 54.4 | 86.7 | 94.5 | 45.7 | 66.3 | 49.4 | 59.3 | 47.3 | 24.6 | 71.0 | 56.0 |
| Ad5-rsv-DPI | 107.0 | 61.7 | 76.7 | 41.7 | 46.7 | 56.7 | 66.8 | 52.5 | 43.8 | 89.6 | 74.1 | 58.8 | 44.6 | 34.3 | 23.8 | 20.1 | 41.5 | 38.6 |
| Ad5-rsv-i.m. | 0 | 0 | 0 | 1.7 | 1.7 | 0 | 8.3 | 1.7 | 0 | 0 | 3.3 | 0 | 0 | 0 | 3.3 | 0 | 1.7 | 0 |
| Ad26-rsv-inh-NA | 183.4 | 66.7 | 126.7 | 170.0 | 208.4 | 78.3 | 185.8 | 191.2 | 147.5 | 107.0 | 133.4 | 173.3 | 77.5 | 118.9 | 109.8 | 94.6 | 83.0 | 126.7 |
| Ad26-rsv-inh-OU | 238.7 | 164.4 | 228.4 | 78.2 | 148.1 | 179.0 | 231.7 | 168.4 | 280.0 | 153.3 | 343.4 | 238.4 | 110.8 | 86.7 | 122.2 | 138.5 | 59.6 | 99.6 |
| Ad26-rsv-NA | 112.8 | 86.9 | 130.8 | 81.9 | 115.9 | 153.8 | 103.3 | 113.0 | 92.5 | 175.7 | 139.2 | 118.3 | 66.3 | 85.1 | 82.3 | 59.1 | 69.7 | 95.3 |
| Ad26-rsv-DPI | 45.1 | 135.3 | 65.2 | 113.9 | 74.2 | 51.5 | 102.2 | 56.6 | 93.5 | 88.3 | 67.5 | 73.1 | 21.3 | 20.6 | 35.7 | 37.0 | 25.2 | 17.1 |
| Ad26-rsv-i.m. | 0 | 5 | 0 | 3.3 | 0 | 1.7 | 3.3 | 1.7 | 1.7 | 1.7 | 1.7 | 1.7 | 0 | 0 | 1.7 | 0 | 1.7 | 0 |
| NS | 0 | 0 | 0 | 0 | 0 | 0 | 0 | 0 | 0 | 0 | 0 | 0 | 0 | 0 | 0 | 0 | 0 | 0 |

Data analysis of groups 1-5: For recombinant adenovirus sAd36-rsv-F, groups 1-4 received sAd36-rsv-F transmucosal formulations, and group 5 received the sAd36-rsv-F injection. The dose was 5 × 10⁷ IFU for the transmucosal formulation and 1 × 10⁸ IFU for the injection. The dose of the transmucosal formulation was halved. The TNF-α, IFN-y, and IL-2 response levels of BALF CD4+ and CD8+ T cells in group 5 were almost undetectable, and the TNF-α, IFN-y, and IL-2 response levels of BALF CD4+ and CD8+ T cells in groups 1-4 were significantly higher than those of group 5. The comparison of groups 1-4 shows that the TNF-α, IFN-γ, and IL-2 response levels of CD4+ and CD8+ T cells induced by different administration routes of the transmucosal formulation were different, specifically, oral aerosol inhalation > nasal aerosol inhalation > nasal drop > oral dry powder inhalation. The preferred administration routes of the transmucosal formulation are oral aerosol inhalation or nasal aerosol inhalation.

Data analysis of groups 6-10: For recombinant adenovirus Ad5-rsv-F, groups 6-9 received Ad5-rsv-F transmucosal formulations, and group 10 received the Ad5-rsv-F injection. The dose was 5 × 10⁷ IFU for the transmucosal formulation and 1 × 10⁸ IFU for the injection. The dose of the transmucosal formulation was halved. The TNF-α, IFN-y, and IL-2 response levels of BALF CD4+ and CD8+ T cells in group 10 were almost undetectable, and the TNF-α, IFN-y, and IL-2 response levels of BALF CD4+ and CD8+ T cells in groups 6-9 were significantly higher than those of group 10. The comparison of groups 6-9 shows that the TNF-α, IFN-γ, and IL-2 response levels of CD4+ and CD8+ T cells induced by different administration routes of the transmucosal formulation were different, specifically, oral aerosol inhalation > nasal aerosol inhalation > nasal drop > oral dry powder inhalation, suggesting that the preferred administration routes of the transmucosal formulation are oral aerosol inhalation or nasal aerosol inhalation. Data analysis of groups 11-15: For recombinant adenovirus Ad26-rsv-F, groups 11-14 received Ad26-rsv-F transmucosal formulations, and group 15 received the Ad26-rsv-F injection. The dose was 5 × 10⁷ IFU for the transmucosal formulation and 1 × 10⁸ IFU for the injection. The dose of the transmucosal formulation was halved. The TNF-α, IFN-y, and IL-2 response levels of BALF CD4+ and CD8+ T cells in group 15 were almost undetectable, and the TNF-α, IFN-γ, and IL-2 response levels of BALF CD4+ and CD8+ T cells in groups 11-14 were significantly higher than those of group 15. The comparison of groups 11-14 shows that the TNF-α, IFN-γ, and IL-2 response levels of CD4+ and CD8+ T cells induced by different administration routes of the transmucosal formulation were different, specifically, oral aerosol inhalation > nasal aerosol inhalation > nasal drop > oral dry powder inhalation. The preferred administration routes of the transmucosal formulation are oral aerosol inhalation or nasal aerosol inhalation.

Based on the data of groups 5, 10, and 15, the injection is difficult to induce the mucosal immune response in the body. The data of groups 1-4, 6-9, and 11-14 show that compared with the injection, the transmucosal formulation induced significantly improved mucosal immune response in the body even if the administration dose was halved. Also, the comparison with the TNF-α, IFN-y, and IL-2 response levels of splenic CD4+ and CD8+ T cells described above indicates that the mucosal immune response synergistically induces the cellular immune response in the body, suggesting that the transmucosal formulation of the present disclosure has outstanding advantages in inducing the mucosal immune response in the body and can synergistically improve the cellular immune response and improve the immunity in the body.

### Example 7. Evaluation of aerosol inhalant dose of transmucosal formulation

Animal study: BALB/c mice were quarantined according to the Standard Operation Procedures for Quarantine of Experimental Animals (SOP-QCM-050), and 90 qualified mice were selected and randomized into 15 groups.

The transmucosal formulation of sAd36-rsv-F, Ad5-rsv-F, and Ad26-rsv-F prepared in Example 4 was administered by oral aerosol inhalation, and the doses of the transmucosal formulation were compared and evaluated.

### Oral aerosol inhalation:

Immunization procedures: The mice were anesthetized with isoflurane; after at least 20 minutes, the mice were fixed on an operating platform at an angle of about 45° to the benchtop, and the transmucosal formulation prepared above was administered through the mouth into the trachea via an aerosol inhalation device; the mouse head was kept in a higher position for 10-20 s before the mouse was returned to the cage. The mice were kept warm by using an infrared lamp until the mice were awake.

| **Group** | **Virus** | **Dose/mouse** | | **Route of administration** | **Number of mice** |
|---|---|---|---|---|---|
| 1 | sAd36-rsv-F | 5×10⁸ IFU | hig-2 | **Oral aerosol inhalation** | 6 |
| 2 | | 1×10⁸ IFU | hig-1 | | 6 |
| 3 | | 5×10⁷ IFU | mid-2 | | 6 |
| 4 | | 1×10⁶ IFU | mid-1 | | 6 |
| 5 | | 0.5×10⁶ IFU | low | | 6 |
| 6 | Ad5-rsv-F | 5×10⁸ IFU | hig-2 | **Oral aerosol inhalation** | 6 |
| 7 | | 1×10⁸ IFU | hig-1 | | 6 |
| 8 | | 5×10⁷ IFU | mid-2 | | 6 |
| 9 | | 1×10⁶ IFU | mid-1 | | 6 |
| 10 | | 0.5×10⁶ IFU | low | | 6 |
| 11 | Ad26-rsv-F | 5×10⁸ IFU | hig-2 | **Oral aerosol inhalation** | 6 |
| 12 | | 1×10⁸ IFU | hig-1 | | 6 |
| 13 | | 5×10⁷ IFU | mid-2 | | 6 |
| 14 | | 1×10⁶ IFU | mid-1 | | 6 |
| 15 | | 0.5×10⁶ IFU | low | | 6 |

### Results:

Blood samples were collected on day 28 to determine the titer of IgG-binding antibody against pre-F by the same method as in Example 5.

**Table 7. Serum titer of IgG binding antibody 28 days after mice were immunized with different doses of sAd36-rsv-F/Ad26-rsv-F/Ad5-rsv-F transmucosal formulation**

| **Group** | **Mouse 1** | **Mouse 1** | **Mouse 1** | **Mouse 1** | **Mouse 1** |
|---|---|---|---|---|---|
| sAd36-rsv-low | 3.49 | 3.8 | 4.1 | 4.4 | 4.1 |
| sAd36-rsv-mid-1 | 4.4 | 4.7 | 5 | 5.3 | 4.4 |
| sAd36-rsv-mid-2 | 5.6 | 6.2 | 6.51 | 5.6 | 6.2 |
| sAd36-rsv-hig-1 | 6.2 | 6.2 | 5.6 | 5.9 | 6.51 |
| sAd36-rsv-high-2 | 6.51 | 6.2 | 6.51 | 5.9 | 5.6 |
| Ad5-rsv-low | 4.4 | 3.49 | 3.8 | 3.8 | 4.1 |
| Ad5-rsv-mid-1 | 5.6 | 5.3 | 5.6 | 5.3 | 5.3 |
| Ad5-rsv-mid-2 | 5.9 | 6.2 | 6.2 | 5.6 | 6.51 |
| Ad5-rsv-high-1 | 6.51 | 6.2 | 5.6 | 6.2 | 6.51 |
| Ad5-rsv-high-2 | 5.9 | 6.51 | 6.2 | 5.9 | 6.51 |
| Ad26-rsv-low | 3.8 | 4.1 | 4.1 | 3.8 | 3.49 |
| Ad26-rsv-mid-1 | 5.3 | 5.6 | 5.3 | 5.3 | 5.6 |
| Ad26-rsv-mid-2 | 5.9 | 6.2 | 6.51 | 6.2 | 5.6 |
| Ad26-rsv-high-1 | 6.51 | 5.6 | 6.2 | 6.2 | 6.51 |
| Ad26-rsv-high-2 | 6.2 | 6.51 | 6.2 | 6.2 | 6.51 |

The results show that, for sAd36-rsv-F, Ad26-rsv-F, and Ad5-rsv-F, the titers of binding antibody and neutralizing antibody generated in the body were gradually increased along with the increase of the dose at the start and entered a plateau stage when the dose reached a certain level. The preferred dose range is between mid-1 and high-1, i.e., 1 × 10⁶-1 × 10⁸ IFU.

Blood samples were collected on day 28 to determine the titer of neutralizing antibody against RSV A2 virus.

**Table 8. Serum titer of neutralizing antibody against RSV A2 virus 28 days after mice were immunized with different doses of sAd36-rsv-F/Ad26-rsv-F/Ad5-rsv-F transmucosal formulation**

| Group | Titer of neutralizing antibody against virus | | | | | |
|---|---|---|---|---|---|---|
| | Mouse 1 | Mouse 2 | Mouse 3 | Mouse 4 | Mouse 5 | Mouse 6 |
| sAd36-rsv-low | 337 | 453 | 343 | 295 | 375 | 498 |
| sAd36-rsv-mid-1 | 705 | 346 | 660 | 599 | 646 | 620 |
| sAd36-rsv-mid-2 | 864 | 869 | 343 | 904 | 958 | 780 |
| sAd36-rsv-hig-1 | 873 | 665 | 921 | 705 | 948 | 825 |
| sAd36-rsv-high-2 | 682 | 931 | 765 | 803 | 885 | 964 |
| Ad5-rsv-low | 371 | 405 | 479 | 258 | 522 | 530 |
| Ad5-rsv-mid-1 | 437 | 604 | 714 | 578 | 450 | 868 |
| Ad5-rsv-mid-2 | 705 | 1007 | 910 | 490 | 992 | 770 |
| Ad5-rsv-high-1 | 582 | 805 | 952 | 771 | 660 | 1157 |
| Ad5-rsv-high-2 | 1098 | 637 | 524 | 648 | 1254 | 937 |
| Ad26-rsv-low | 358 | 333 | 276 | 301 | 416 | 295 |
| Ad26-rsv-mid-1 | 440 | 350 | 477 | 783 | 849 | 489 |
| Ad26-rsv-mid-2 | 757 | 687 | 758 | 470 | 898 | 1095 |
| Ad26-rsv-high-1 | 582 | 805 | 952 | 771 | 1157 | 606 |
| Ad26-rsv-high-2 | 691 | 771 | 944 | 957 | 885 | 729 |

For sAd36-rsv-F, Ad26-rsv-F, and Ad5-rsv-F, the titers of binding antibody and neutralizing antibody generated in the body were gradually increased along with the increase of the dose at the start and entered a plateau stage when the dose reached a certain level. The preferred dose range is between mid-1 and high-1, i.e., 1 × 10⁶-1 × 10⁸ IFU. The optimal dose in humans is 1 × 10⁷-1.3 × 10⁹ IFU, as calculated according to the equivalent dose ratios based on the surface area between humans and animals, as directed by classical textbooks such as *Pharmacological*

### Experimental Methodology.

### Example 8. Evaluation of immune response of transmucosal formulations with various immunogens

Animal study: BALB/c mice were quarantined according to the Standard Operation Procedures for Quarantine of Experimental Animals (SOP-QCM-050), and 30 qualified mice were selected and randomized into 6 groups.

The transmucosal formulation prepared in Example 4 was administered for immunization according to the following administration routes and doses.

Oral aerosol inhalation: Immunization procedures: The mice were anesthetized with isoflurane; after at least 20 minutes, the mice were fixed on an operating platform at an angle of about 45° to the benchtop, and the transmucosal formulation prepared above was administered through the mouth into the trachea via an aerosol inhalation device; the mouse head was kept in a higher position for 10-20 s before the mouse was returned to the cage. The mice were kept warm by using an infrared lamp until the mice were awake.

| **Group** | **Virus** | **Route of administration** | **Number of immunizations** | **Dose/mouse** |
|---|---|---|---|---|
| Group 1 | sAd36-rsv-F | Aerosol inhalation | 5 mice | 5×10⁷ IFU |
| Group 2 | sAd36-rsv-F/G | | 5 mice | 5×10⁷ IFU |
| Group 3 | Ad5-rsv-F | | 5 mice | 5×10⁷ IFU |
| Group 4 | Ad5-rsv-F/G | | 5 mice | 5×10⁷ IFU |
| Group 5 | Ad26-rsv-F | | 5 mice | 5×10⁷ IFU |
| Group 6 | Ad26rsv-F/G | | 5 mice | 5×10⁷ IFU |

### Results

Blood samples were collected on days 28 and 42 to determine the titer of IgG binding antibody against pre-F by the same method as in Example 5

**Table 9. Titer of IgG binding antibody 28 days and 42 days after immunization with different immunogen transmucosal formulations**

| **Group** | **D28** | | | | | **D42** | | | | |
|---|---|---|---|---|---|---|---|---|---|---|
| | Mouse 1 | Mouse 2 | Mouse 3 | Mouse 4 | Mouse 5 | Mouse 1 | Mouse 2 | Mouse 3 | Mouse 4 | Mouse 5 |
| sAd36-rsv-inh (Pre-F/G) | 5.01 | 5.31 | 5.61 | 5.91 | 5.61 | 5.01 | 5.01 | 4.41 | 4.71 | 5.61 |
| sAd36-rsv-inh (Pre-F) | 5.61 | 5.31 | 5.01 | 5.31 | 5.31 | 4.41 | 4.71 | 4.41 | 5.01 | 4.71 |
| Ad5-rsv-inh (Pre-F/G) | 5.61 | 5.01 | 5.31 | 5.61 | 5.91 | 5.31 | 4.71 | 5.01 | 4.71 | 5.01 |
| Ad5-rsv-inh (Pre-F) | 5.01 | 5.61 | 5.31 | 5.61 | 5.91 | 5.01 | 4.11 | 4.71 | 5.31 | 5.01 |
| Ad26-rsv-inh (Pre-F/G) | 5.01 | 5.31 | 5.61 | 5.31 | 5.91 | 4.71 | 5.01 | 4.11 | 4.71 | 5.31 |
| Ad26-rsv-inh (Pre-F) | 5.61 | 4.71 | 5.01 | 5.31 | 5.61 | 4.11 | 4.11 | 4.71 | 5.01 | 5.31 |

Blood samples were collected on days 28 and 42 to determine the titer of neutralizing antibody against RSV A2 virus by the same method as in Example 5

**Table 10. Titer of neutralizing antibody against RSV A2 virus 28 days and 42 days after immunization with different immunogen transmucosal formulations**

| **Group** | **D28** | | | | | **D42** | | | | |
|---|---|---|---|---|---|---|---|---|---|---|
| | **Mouse 1** | **Mouse 2** | **Mouse 3** | **Mouse 4** | **Mouse 5** | **Mouse 1** | **Mouse 2** | **Mouse 3** | **Mouse 4** | **Mouse 5** |
| sAd36-rsv-inh (Pre-F/G) | 1138 | 1429 | 1234 | 1031 | 1248 | 579 | 647 | 730 | 715 | 688 |
| sAd36-rsv-inh (Pre-F) | 608 | 795 | 979 | 843 | 832 | 458 | 538 | 693 | 552 | 417 |
| Ad5-rsv-inh (Pre-F/G) | 1271 | 1180 | 992 | 1073 | 1156 | 814 | 1050 | 994 | 805 | 916 |
| Ad5-rsv-inh (Pre-F) | 662 | 950 | 776 | 698 | 893 | 410 | 386 | 354 | 224 | 483 |
| Ad26-rsv-inh (Pre-F/G) | 1051 | 979 | 1178 | 1105 | 968 | 714 | 661 | 698 | 549 | 638 |
| Ad26-rsv-inh (Pre-F) | 893 | 835 | 768 | 540 | 683 | 357 | 442 | 349 | 410 | 337 |

The data show that both immunogens F or F/G can induce the humoral immune response in the body and produce a high titer of neutralizing antibody. By comparing the data of groups 1, 3, and 5 with groups 2, 4, and 6, it is indicated that the titer of IgG binding antibody and the titer of neutralizing antibody against pre-F in the body are slightly higher than those of the immunogen F when the immunogen is F/G. The identity of the immunogen does not affect the advantages of the transmucosal formulation.

The above description is only for the purpose of illustrating the preferred examples of the present disclosure, and is not intended to limit the scope of the present disclosure. Any modifications, equivalents, and the like made without departing from the spirit and principle of the present disclosure shall fall in the claimed scope of the present disclosure.

The foregoing examples and methods described herein may vary based on the abilities, experience, and preferences of those skilled in the art. Certain sequence of the steps of methods listed in the present disclosure does not constitute any limitation on the sequence of steps of methods encompassed by the present disclosure.

## Claims

1. A composition for resisting respiratory syncytial virus (RSV) infection, comprising a recombinant adenovirus vector, wherein a gene encoding an RSV antigen is inserted into the recombinant adenovirus vector, and wherein the composition is a transmucosal formulation.

2. The composition according to claim 1, wherein the adenovirus vector comprises a human adenovirus and/or a simian adenovirus vector; preferably, the human adenovirus is selected from Ad5, Ad26, Ad35, and Ad48, and the simian adenovirus vector is selected from sAd36 and sAd37; more preferably, the recombinant adenovirus is preferably Ad5, Ad26, or sAd36.

3. The composition according to claim 1, wherein the gene encoding the RSV antigen is a gene encoding one or more of RSV F protein of the prefusion conformation (pre-F), attachment protein G, and/or small hydrophobic (SH) protein.

4. The composition according to claim 1, wherein the transmucosal formulation is an aerosol inhalation formulation, a nasal drop or spray formulation, or a dry powder inhalation formulation; preferably, the aerosol inhalation formulation is an oral aerosol inhalation formulation or a nasal aerosol inhalation formulation.

5. The composition according to claim 1, wherein the gene encoding the RSV antigen is derived from a native RSV strain or a recombinant RSV strain, preferably from a human RSV strain, and more preferably the A2, Long, or B strain.

6. The composition according to claim 1, comprising a pharmaceutically acceptable excipient, wherein preferably, the pharmaceutically acceptable excipient is selected from one or more of: a buffer, a protectant, a stabilizer, a surfactant, an osmotic pressure regulator, a preservative, an inactivator, and a human albumin; more preferably, the human albumin is human serum albumin; and/or, the buffer is selected from one or more of HEPES, HIS, TRIS, PB, succinate, and citrate; and/or, the protectant is selected from one or more of gelatin, ethanol, ethylenediamine tetraacetate (EDTA), disodium edtate (EDTA-2Na), and magnesium chloride; and/or, the stabilizer is selected from one or more of sucrose, mannitol, fucose, and maltose; and/or the surfactant is selected from one or more of Tween, Span, and glycerol; and/or, the osmotic pressure regulator is selected from sodium chloride; more preferably, the excipient component comprises mannitol, sucrose, sodium chloride, magnesium chloride, HEPES, polysorbate 80, and glycerol; more preferably, the excipient component comprises 5-300 mg/mL of mannitol, 0.1-30 mg/mL of sodium chloride, 0.05-10 mg/mL of HEPES, 0.01-10 mg/mL of polysorbate 80, 0.1-5 mg/mL of glycerol, 0.1-5 mg/mL of magnesium chloride, and 1-80 mg/mL of sucrose; more preferably, the excipient component comprises 10-50 mg/mL of mannitol, 1-10 mg/mL of sodium chloride, 0.1-5 mg/mL of HEPES, 0.1-5 mg/mL of polysorbate 80, 0.5-5 mg/mL of glycerol, 0.1-5 mg/mL of magnesium chloride, and 5-30 mg/mL of sucrose.

7. The composition according to claim 1, wherein the dose of the recombinant adenovirus in the transmucosal formulation is 1 × 10⁷ to 1 × 10¹⁰ IFU/dose, preferably 1 × 10⁷ to 6 × 10⁹ IFU/dose, and more preferably 1 × 10⁷ to 1.3 × 10⁹ IFU/dose.

8. The composition according to claim 1, wherein the unit dose of the formulation is 0.05-5 mL, preferably 0.1-1 mL.

9. A method of preparing the composition according to any one of claims 1-8, comprising: propagating the recombinant adenovirus in a host cell culture, isolating and purifying the recombinant adenovirus vector, and formulating the recombinant adenovirus vector into the transmucosal formulation.

10. The method according to claim 9, wherein the recombinant adenovirus vector lacks at least a portion of the E1 region and/or the E3 region of the adenovirus genome.

11. The method according to claim 10, wherein the recombinant adenovirus lacks at least a portion of the E4 region and/or the E2 region of the adenovirus genome.

12. Use of the composition according to any one of claims 1-8 in preparing a vaccine for inducing an immune response in a mammal, wherein preferably, the immunization is a prime immunization or a boost immunization.

13. The use according to claim 12, wherein the composition is administered in one dose, two doses, or three doses.

14. The use according to claim 12, wherein the composition is administered in combination with an immunological composition of a recombinant adenovirus of an identical or different serotype, and/or,
in combination with a composition of a protein expressed by an RSV antigenic nucleic acid, and/or,
in combination with an adjuvant, and/or,
in combination with an additional therapeutic agent;
preferably, the additional therapeutic agent is selected from a therapeutic agent against RSV infection, more preferably, the additional therapeutic agent is selected from a standard therapeutic agent against lower respiratory tract RSV infection during hospitalization; more preferably, the additional therapeutic agent is selected from one or more of a bronchodilator, an antibiotic, epinephrine, an anticholinergic, an antipyretic, and a nonsteroid anti-inflammatory drug.

15. A kit for preventing and/or treating respiratory syncytial virus infection, comprising the transmucosal formulation according to any one of claims 1-8 and a delivery device.

16. The kit according to claim 15, wherein the delivery device is an aerosol inhalation device, preferably an aerosol generator.

17. The kit according to claim 16, wherein the vaccine is atomized by the aerosol inhalation device to form particles with a size of 10 µm or less, preferably 0.5 to 10 µm, and more preferably 5 to 10 µm.
